# FASCICULE DE BREVET EUROPEEN

(11) **EP 0 684 241 B1**
(45) Date de publication et mention de la délivrance du brevet: **27.08.1997**
(21) Numéro de dépôt: 95401194.6
(22) Date de dépôt: 23.05.1995
(51) Int. Cl.: C07D 409/12, C07D 405/12, C07D 401/12, A61K 31/44, A61K 31/495

(54) **N-pyridyl carboxamides et dérivés, leurs procédés de préparation et les compositions pharmaceutiques qui les contiennent**
N-pyridyl Carboxamidderivate, Verfahren zu ihrer Herstellung und diese enthaltende pharmazeutischen Zusammensetzungen
N-pyridyl carboxamide derivatives, processes for their preparation and pharmaceutical compositions containing them

(30) Priorité: 27.05.1994 FR 9406412
(43) Date de publication de la demande: 29.11.1995
(73) Titulaire: ADIR ET COMPAGNIE, 92415 Courbevoie Cédex (FR)
(72) Inventeur: Robert, Jean-Michel, F-44200 Nantes (FR); Rideau, Odile, F-44000 Nantes (FR); Robert-Piessard, Sylvie, F-44200 Nantes (FR); Courant, Jacqueline, F-44300 Nantes (FR); Le Baut, Guillaume, F-44230 Saint Sebastien sur Loire (FR); Caignard, Daniel-Henri, F-75015 Paris (FR); Renard, Pierre, F-78000 Versailles (FR); Adam, Gérard, F-78600 Le Mesnil le Roi (FR)

(56) Documents cités:
- EP-A- 0 465 913
- CA 122:68755T

## Description

La présente invention concerne de nouveaux N-pyridylcarboxamides et dérivés, leurs procédés de préparation et les compositions pharmaceutiques qui les contiennent.

Des structures N-pyridylcarboxamides sont déjà décrites. Ainsi la demande de brevet WO 9304580 décrit des N-(4-pyridyl)arylacétamides comme pesticides.

On peut également citer le brevet EP 465913 concernant des (2,3)-diamino-(5)-trifluorométhylpyridines ayant une activité inhibitrice de la phospholipase A₂ inhérente à la présence du groupement CF₃ en position 5.

La demanderesse a présentement découvert que de nouveaux dérivés N-pyridylcarboxamides étaient des dérivés atoxiques doués de propriétés antiinflammatoires et/ou diurétiques de haut niveau. L'activité antiinflammatoire des dérivés de l'invention présente la caractéristique particulièrement avantageuse de se manifester après administration par voie systémique, mais également par voie topique ce qui rend, outre les indications classiques des antiinflammatoires, les composés de l'invention particulièrement intéressants dans des maladies cutanées comme le psoriasis. En outre, la composante diurétique de certains produits de l'invention les rend très intéressants dans certaines maladies inflammatoires rénales.

Plus spécifiquement l'invention concerne les dérivés de formule (I): 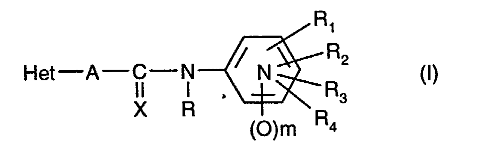 dans laquelle
m vaut 0 ou 1, le symbole 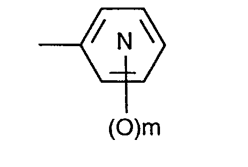 représentant lorsque m vaut 0, le noyau pyridine, et, lorsque m vaut 1, le N-oxyde de la pyridine,
   le système pyridinique 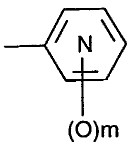 étant fixé au groupement 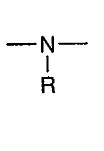 qui le porte soit en position 2, soit en position 3 de la pyridine ;
R₁, R₂, identiques ou différents, sont choisis, chacun indépendamment l'un de l'autre, parmi hydrogène, amino, alkylamino, dialkylamino, alkyle, hydroxy, alkoxy, nitro et halogène,
R₃, R₄, identiques ou différents, sont choisis chacun indépendamment l'un de l'autre parmi amino, alkylamino, dialkylamino, alkyle, hydroxy, alkoxy, nitro et halogène,
R représente un atome d'hydrogène ou un groupement alkyle,
A représente une liaison simple ; et dans ce cas Het représente un groupement choisi parmi pyrazine, pyrazine substituée (à l'exception du N-(4,6-diméthylpyridin-2-yl)-(5-méthylpyrazin-2-yl)carboxamide), benzothiophène, benzothiophène substitué, 4-oxo[4H] benzopyrane, 4-oxo[4H]benzopyrane substitué, pyrrole, pyrrole substitué, pyrroline, pyrroline substituée, pyrrolidine, pyrrolidine substituée, pipéridine, pipéridine substituée, pyridine, pyridine substituée, benzopyrane, benzopyrane substitué par un ou plusieurs groupements alkyle, chromane, chromane substitué par un ou plusieurs groupements alkyle, 3-carboxy-5-alkyl-isoxazole, 3-alkoxycarbonyl-5-alkyl-isoxazole, phtalimido et phtalimido substitué,
ou bien A représente un groupement alkylène non substitué ou substitué par un ou plusieurs groupements alkyles, ou un groupement alcénylène non substitué ou substitué par un ou plusieurs groupements alkyle ; et dans ce cas Het représente un groupement choisi parmi thiophène, thiophène substitué, pyrazine, pyrazine substituée, benzothiophène, benzothiophène substitué, 4-oxo[4H]benzopyrane, 4-oxo[4H]benzopyrane substitué, pyrrole, pyrrole substitué, pyrroline, pyrroline substituée, pyrrolidine, pyrrolidine substituée, pipéridine, pipéridine substituée, pyridine, pyridine substituée, benzopyrane, benzopyrane substitué par un ou plusieurs groupements alkyle, chromane, chromane substitué par un ou plusieurs groupements alkyle, 3-carboxy-5-alkyl-isoxazole, 3-alkoxycarbonyl-5-alkyl-isoxazole, phtalimido et phtalimido substitué,
X représente un atome d'oxygène, un atome de soufre, un groupement imino ou un groupement imino substitué par un groupement choisi parmi alkyle, alkoxy, hydroxy, amino, arylalkyloxy et aryloxy,
leurs énantiomères et diastéréoisomères et leurs sels d'addition à un acide ou une base pharmaceutiquement acceptable,
étant entendu que, sauf précisions contraires :
- le terme "substitué" affectant les systèmes thiophène, pyrazine, benzothiophène, 4-oxo[4H]benzopyrane, pyrrole, pyrroline, pyrrolidine, pipéridine, pyridine et phtalimido, signifie que ces systèmes sont substitués par un ou plusieurs groupements choisis parmi alkyle, alkoxy, trifluorométhyle, hydroxy, halogène, thiol et alkylthio,
- les termes "alkyle" et "alkoxy", "alkylène", désignent des groupements linéaires ou ramifiés comprenant de 1 à 6 atomes de carbone,
- le terme "aryle" désigne un radical phényle ou naphthyle,
- le terme "alcénylène" désigne une chaîne insaturée linéaire ou ramifiée contenant de 2 à 6 atomes de carbone.

Parmi les acides pharmaceutiquement acceptables que l'on peut ajouter aux composés de formule (I) pour obtenir un sel, on peut citer, à titre non limitatif, les acides chlorhydrique, sulfurique, tartrique, maléique, fumarique, oxalique, méthanesulfonique et camphorique.

Parmi les bases pharmaceutiquement acceptables que l'on peut utiliser pour salifier les composés de l'invention on peut citer à titre d'exemples non exhaustifs la soude, la potasse, la triéthylamine, la diéthylamine, l'éthanolamine ou la diéthanolamine, l'arginine et la lysine.

L'invention concerne particulièrement les composés de formule (I) dans lesquels, pris ensemble ou séparément,
- le symbole 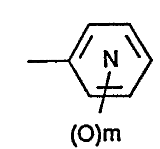 représente un groupement pyridin-2-yle,
- le symbole 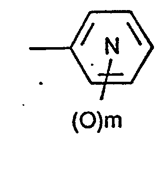 représente un groupement pyridin-3-yle,
- deux des substituants R₁, R₂, R₃ et R₄ représentent un radical méthyle,
- R représente un hydrogène,
- X représente un souffre,
- X représente un groupement imino,
- X représente un groupement imino substitué par un groupement hydroxy, un groupement méthoxy, un groupement méthyle ou un groupement amino,
- A représente un méthylène,
- Het représente un groupement thiophène ou thiophène substitué,
- Het représente un groupement pyrazine ou pyrazine substitué,
- Het représente un groupement benzothiophène ou benzothiophène substitué,
- Het représente un groupement 4-oxo[4H]benzopyrane ou 4-oxo[4H]benzopyrane substitué,
- Het représente un groupement pyrrole ou pyrrole substitué,
- Het représente un groupement pyrrolidine ou pyrrolidine substitué,
- Het représente un groupement pyridine ou pyridine substitué,
- Het représente un groupement phtalimido ou phtalimido substitué,
- Het représente un groupement 3-carboxy-5-méthyl-isoxazole,
- et Het représente un groupement 3-éthoxycarbonyl-5-méthyl-isoxazole.

Des cas particuliers de l'invention concernent par exemple :
. les composés ayant la formule (IA) : 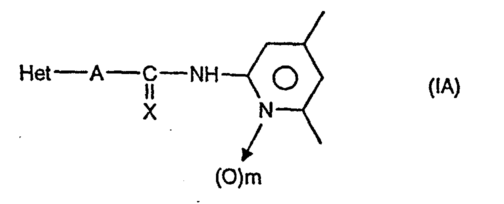 dans laquelle m représente 0 ou 1, et
   - soit Het représente le groupement thiophène et A représente un méthylène,
   - soit Het représente le groupement pyrazine ou le groupement pyrazine substitué par un groupement alkyle et A est une liaison simple,
   et X représente un atome d'oxygène, un atome de soufre, un groupement imino ou un groupement imino substitué par un groupement hydroxy, un groupement méthoxy, un groupement méthyle, un groupement amino, ou un groupement benzyloxy, leurs énantiomères et diastéréoisomères et leurs sels d'addition à un acide pharmaceutiquement acceptable,
   - le composé qui est le N-(4,6-diméthyl-pyridin-2-yl)-(thién-2-yl)acétamide, son N-oxyde et ses sels d'addition à un acide pharmaceutiquement acceptable,
   - le composé qui est le N-(4,6-diméthyl-pyridin-2-yl)-(thién-3-yl)acétamide, son N-oxyde et ses sels d'addition à un acide pharmaceutiquement acceptable,
   - le composé qui est la N-(4,6-diméthyl-pyridin-2-yl)-(pyrazin-2-yl)carbamidoxime de formule 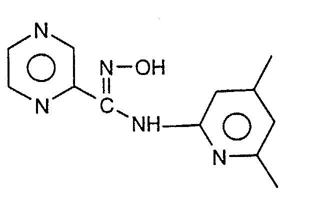 son N-oxyde, et ses sels d'addition à un acide pharmaceutiquement acceptable,
   - le composé qui est la N-(4,6-diméthyl-pyridin-2-yl)-O-méthyl-(pyrazin-2-yl) carbamidoxime de formule 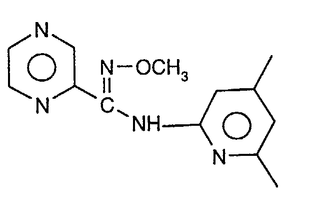 son N-oxyde, et ses sels d'addition à un acide pharmaceutiquement acceptable,
   - le composé qui est le N-(4,6-diméthyl-pyridin-2-yl)-(5-méthyl-pyrazin-2-yl)thiocarboxamide, son N-oxyde et ses sels d'addition à un acide pharmaceutiquement acceptable,
   - le composé qui est le N-(4,6-diméthyl-pyridin-2-yl)-(pyrazin-2-yl)carboxamidine, son N-oxyde, et ses sels d'addition à un acide pharmaceutiquement acceptable,
   - le composé qui est le N-méthyl-N'-(4,6-diméthyl-pyridin-2-yl)-(pyrazin-2-yl)carboxamidine 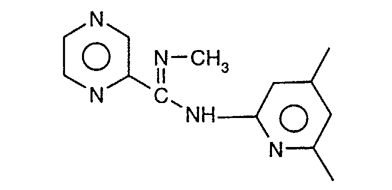 son N-oxyde, et ses sels d'addition à un acide pharmaceutiquement acceptable.

La présente invention a également pour objet le procédé de préparation des composés de formule (I) caractérisé en ce que l'on utilise comme matière première un dérivé de formule (1): 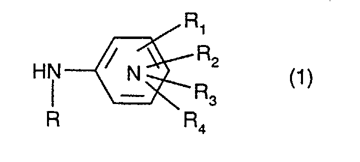 où R, R₁, R₂, R₃, R₄ et le cycle 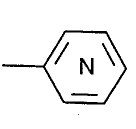 ont la même définition que dans la formule (I),
- soit que l'on condense avec un dérivé de formule (2) :

   Het-A-COOH (2)

   où Het et A ont la même définition que dans la formule (I), pour conduire à un dérivé de formule (I/a) : 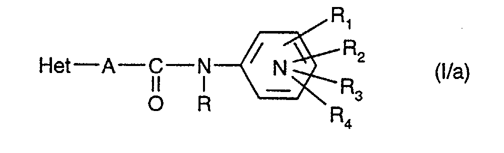 où Het, A, R, R₁, R₂, R₃ et R₄ et le cycle 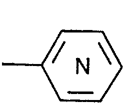 ont la même définition que précédemment, dérivé de formule (I/a), qui est soumis à un agent de thionation pour conduire à un dérivé de formule (I/b) : 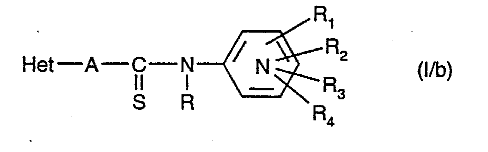 où Het, A, R, R₁, R₂, R₃ et R₄ et le cycle 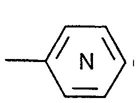 ont la même signification que précédemment, dérivé de formule (I/b), qui est condensé avec un dérivé de formule (3) : 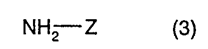 où Z représente un atome d'hydrogène, un groupement hydroxy, alkyle, alkoxy, amino, arylalkyloxy et aryloxy pour conduire à un dérivé de formule (I/c): 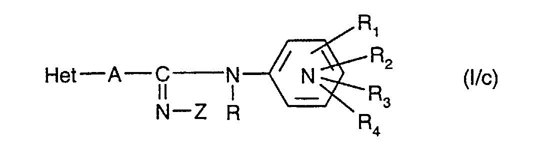 où Het, A, R, R₁, R₂, R₃,R₄, Z et le cycle 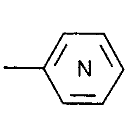 ont la même définition que précédemment,
- soit que l'on fait réagir avec un composé de formule (4) : 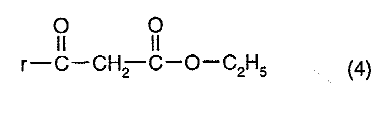 où r est un groupement alkyle,
   afin d'obtenir des composés de formule (5) : 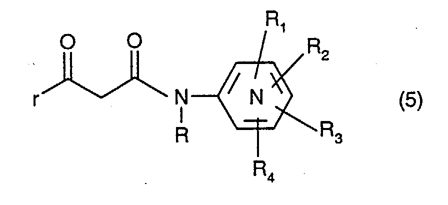 dans laquelle r, R, R₁, R₂, R₃, R₄ sont tels que définis précédemment,
   qui sont mis à réagir avec un composé de formule Het-H où Het est tel que défini dans la formule (I),
   afin d'obtenir des composés de formule (I/d), 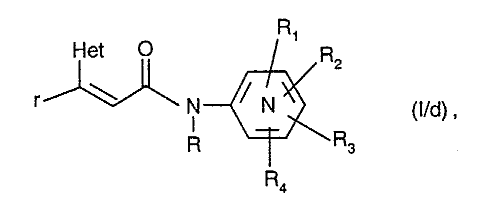 dans laquelle Het, r, R, R₁, R₂, R₃, R₄ sont tels que définis précédemment,
   composés de formule (I/d) qui peuvent, si on le désire, être réduits, par l'action de borohydrure de sodium par exemple, pour donner des composés de formule (I/e) : 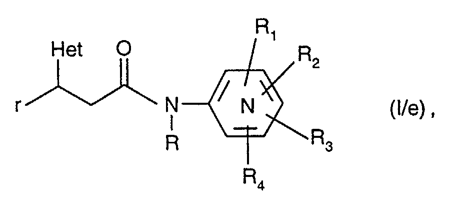 dans laquelle Het, r, R, R₁, R₂, R₃, R₄ sont tels que définis précédemment,
   ou bien qui peuvent, dans le cas où Het représente un groupement pyrrolidine, réagir avec du chlorooximidoacétate d'alkyle de formule (6) : 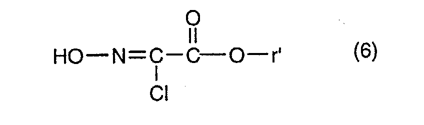 où r' est un groupement alkyle,
   pour donner des composés de formule (I/f) : 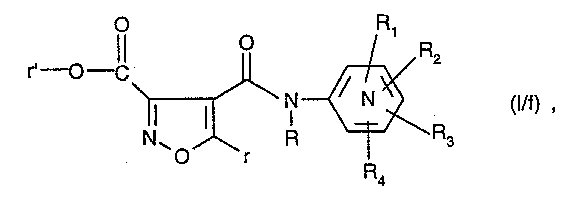 dans laquelle r, r', R, R₁, R₂, R₃, R₄ sont tels que définis précédemment,
   composés de formule (I/f) qui peuvent réagir, si on le désire, avec de l'hydroxyde de lithium, afin d'obtenir des composés de formule (I/g) : 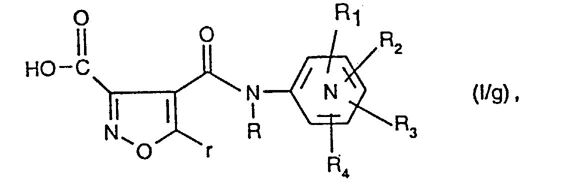 dans laquelle r, R, R₁, R₂, R₃, R₄ sont tels que définis précédemment,
   dérivés de formule (I/a), (I/b), (I/c), (I/d), (I/e), (I/f), (I/g) que l'on peut transformer, si on le désire, en dérivés N-oxydes de la pyridine par action d'eau oxygénée,
   les dérivés de formule (I/a), (I/b), (I/c), (I/d), (I/e), (I/f), (I/g) ainsi que leurs N-oxydes formant l'ensemble des dérivés de formule (I), dérivés de formule (I) dont on peut séparer les énantiomères et diastéréoisomères et que l'on peut salifier par un acide ou une base pharmaceutiquement acceptable.

Les composés de formule (I) possèdent d'intéressantes propriétés pharmacologiques.

L'étude de ces propriétés a en effet montré que les dérivés de formule (I) n'étaient pas toxiques, et étaient doués d'activité antiinflammatoire, qui se manifeste aussi bien par voie topique que systémique, ainsi que d'activité diurétique.

Ce spectre d'activité rend donc les composés de la présente invention utiles dans le traitement de l'arthrite chronique ou aigüe et utiles dans un certain nombre d'indications telles que les rhumatismes inflammatoires, la polyarthrite rhumatoïde, la spondylarthrite ankylosante, les arthroses, les rhumatismes articulaires, les lombalgies. De par leur activité par voie topique les composés de l'invention sont utiles dans le traitement de certains troubles cutanés comme le psoriasis et l'eczéma. En outre, de par leur activité diurétique, les composés de l'invention sont utiles dans le traitement des maladies inflammatoires rénales, des néphrites, des glomérulonéphrites et des pyélonéphrites.

La présente invention a également pour objet les compositions pharmaceutiques contenant un composé de formule (I), ou un de ses sels d'addition à un acide ou une base pharmaceutiquement acceptable, en combinaison avec un ou plusieurs excipients pharmacologiquement acceptables.

Parmi les compositions pharmaceutiques selon l'invention, on pourra citer plus particulièrement, à titre d'exemples et de façon non limitative, celles qui conviennent à l'administration orale, parentérale, nasale, rectale, perlinguale, oculaire, cutanée, transcutanée, percutanée ou pulmonaire et notamment les préparations injectables, les aérosols, les gouttes oculaires ou nasales, les suppositoires, les comprimés simples, pelliculés ou dragéifiés, les gélules, les capsules, les crèmes, pommades, et gels dermiques.

La posologie utile varie selon l'âge, le sexe et le poids du patient, la voie d'administration, la nature de l'affection et des traitements éventuellement associés et s'échelonne entre 1 mg et 5 grammes par 24 heures, de préférence entre 1 mg et 100 mg par 24 h, plus particulièrement entre 1 et 10 mg par 24 h, par exemple 10 mg.

Les exemples qui suivent illustrent l'invention et ne la limitent en aucune façon.

Les spectres infrarouge sont réalisés en pastille de bromure de potassium renfermant environ 1% du produit à analyser.

Les matières premières utilisées sont soit commerciales soit accessibles à l'homme du métier d'après la littérature et d'après les préparations qui ne font pas partie de l'invention mais qui sont utiles pour préparer certains produits de l'invention.

### PRÉPARATION: 5-BROMO-2,3-DIAMINO 4,6-DIMÉTHYLPYRIDINE

### STADE A : 2-AMINO-5-BROMO-4,6-DIMÉTHYLPYRIDINE

Dissoudre 6,1 g (50 mmol) de 2-amino-4,6-diméthylpyridine dans 50 ml d'acide acétique. Ajouter goutte à goutte à l'aide d'une ampoule de coulée une solution de 8 g (50 mmol) de brome dans 50 ml d'acide acétique. Laisser revenir le milieu réactionnel à température ambiante et poursuivre l'agitation pendant 3 heures. Refroidir dans un bain de glace puis ajouter de la soude à 40 % jusqu'à pH basique. Filtrer, sécher. Reprendre par un peu d'éther isopropylique puis chromatographier sur gel de silice en éluant par ce même solvant. Recueillir le produit attendu sous forme de cristaux blancs. Recristalliser dans l'éthanol absolu.
Rendement : 69 %

### STADE B : 2-AMINO-5-BROMO-4,6-DIMÉTHYL-3-NITROPYRIDINE

Dans 16 ml d'acide sulfurique concentré placé sous agitation et refroidi dans de la glace, dissoudre 4g (20 mmol) du produit obtenu au stade A. Porter la solution à 55°C et ajouter goutte à goutte 1,3 ml d'acide nitrique concentré en veillant à maintenir la température entre 55 et 60°C. Poursuivre l'agitation pendant 20 minutes puis verser le mélange sur de la glace pilée. Faire précipiter le produit par addition de soude à 40 %. Filtrer, laver à l'eau puis sécher. Recueillir ainsi 3,7 g de produit. Recristalliser dans de l'éthanol à 95°.
Rendement: 75 %
Point de fusion : 169°C

### STADE C : 5-BROMO-2,3-DIAMINO-4,6-DIMETHYLPYRIDINE

Refroidir dans un bain de glace une solution de 4,1 g (21,6 mmol) de SnCl₂ dans 20 ml d'HCl concentré. Additioner progressivement 1,32 g (5,4 mmol) de produit obtenu au stade B. Chauffer à 80°C pendant 30 minutes. Laisser refroidir puis verser sur de la glace pilée. Alcaliniser par addition de soude. Filtrer le précipité formé, le laver à l'eau puis le sécher. Recueillir ainsi 1,06 g de poudre blanche. Recristalliser dans le toluène.
Rendement : 90 %
Point de fusion : 183°C

### EXEMPLE 1 : N-(4,6-DIMETHYLPYRIDIN-2-YL)-(THIEN-2-YL)ACETAMIDE

Dans 120 ml de tétrahydrofurane, dissoudre 9,22 g de triphénylphosphine, 6,93 ml de trichlorobrométhane, 5 g d'acide (thién-2-yl)acétique et 8,50 g de 2-amino-4,6-diméthylpyridine. Porter au reflux, filtrer et concentrer le filtrat ; chromatographier sur gel de silice en éluant au dichlorométhane et recristalliser le produit obtenu dans l'éther isopropylique.
Rendement : 78 %
Point de fusion : 124 - 125°C
Composition centésimale
Calculée : C 63.33H 5.68N 11.36
Trouvée : C 63.26H 5.69N 11.34
Caractéristiques spectrales
Infrarouge : 3265 cm⁻¹ ν NH
Résonance Magnétique Nucléaire (solvant CDCl₃)
Noyau pyridinique:
4-CH₃ : 2.29 ppm singulet
6-CH₃ : 2.36 ppm singulet
H₃ : 7.88 ppm singulet
H₅ : 6.72 ppm singulet
CH₂-CO : 3.91 ppm singulet

### EXEMPLE 2 : N-ETHYL-N-(4,6-DIMETHYLPYRIDIN-2-YL)-(THIEN-2-YL)ACETAMIDE

Dissoudre 3,4 g d'iodure de 2-chloro-1-méthyl pyridinium dans du chlorure de méthylène puis ajouter :
. 1,89 g d'acide (thién-2-yl)acétique
. 2 g de 2-éthylamino-4,6-diméthylpyridine
. 4,6 ml de triéthylamine

Porter au reflux. La réaction terminée, filtrer, évaporer à sec ; extraire le résidu ; réunir les phases organiques et les sécher . Evaporer et chromatographier le résidu sur un mélange dichlorométhane éthanol. On obtient un produit huileux.
Rendement : 88 %
Indice de réfraction : 1,561
Caractéristiques spectrales :
Résonance Magnétique Nucléaire (solvant CDCl₃)
CH₂-CH₃ : triplet, CH₃, 3H, δ1 : 1.12 ppm
CH₂-CH₃ : quadruplet, CH₂, 2H, δ : 3.80 ppm

### EXEMPLE 3 : N-HEXYL N-(4,6-DIMETHYLPYRIDIN-2-YL)-(THIEN-2-YL)ACETAMIDE

En procédant comme dans l'exemple 2 mais en remplaçant la 2-éthylamino-4,6-diméthylpyridine par la 2-hexylamino 4,6-diméthylpyridine. On obtient le produit du titre.
Rendement : 60 %
Indice de réfraction : 1,547
Caractéristiques spectrales :
Résonance Magnétique Nucléaire ¹H (solvant CDCl₃)
CH₃-CH₂: triplet 3H : 0.83 ppm
CH₃-CH₂-CH₂-CH₂-CH₂-CH₂-N: multiplet 6H : 1.26 ppm
CH₃-CH₂-CH₂-CH₂-CH₂-CH₂-N : multiplet 2H : 1.51 ppm
CH₂-N : triplet 2H : 3.78 ppm.

### EXEMPLE 4 : N-(5-BROMO 4,6-DIMETHYLPYRIDIN-2-YL)-(THIEN-2-YL)ACETAMIDE

En procédant comme dans l'exemple 2 mais en remplaçant la 2-éthylamino-4,6-diméthylpyridine par la 2-amino-5-bromo-4,6-diméthylpyridine obtenue au stade A de la préparation, on obtient le produit du titre.
Solvant de recristallisation : acétone
Rendement : 86 %
Point de fusion : 192°C
Caractéristiques spectrales :
Résonance Magnétique Nucléaire ¹H/(Solvant CDCl₃)
CH₂ : singulet, 2H : 3.94 ppm

### EXEMPLE 5 : N-(3,5-DIBROMO-4,6-DIMETHYLPYRIDIN-2-YL)-(THIEN-2-YL)ACETAMIDE

En procédant comme dans l'exemple 2 mais en remplaçant la 2-éthylamino-4,6-diméthylpyridine par la 2-amino-3,5-dibromo-4,6-diméthylpyridine, on obtient le produit du titre.
Point de fusion : 154°C

### EXEMPLE 6 : N-(3,5-DICHLOROPYRIDIN-2-YL)-(THIEN-2-YL)ACETAMIDE

En procédant comme dans l'exemple 1 mais en remplaçant la 2-amino-4,6-diméthylpyridine par la 2-amino 3,5-dichloropyridine on obtient le produit du titre.
Recristallisation : éther isopropylique
Rendement : 33 %
Point de fusion : 144 - 145°C
Caractéristiques spectrales :
Infra-rouge
3240 cm⁻¹, ν NH
1680 cm⁻¹, ν CO
Résonance Magnétique Nucléaire ¹H(solvant CDCl₃)
CH₂ : singulet 2H : 4.17 ppm

### EXEMPLE 7: N-(2-AMINO-5-BROMO-4,6-DIMETHYLPYRIDIN-3-YL)-(THIEN-2-YL)ACETAMIDE

En procédant comme dans l'exemple 1, mais en remplaçant la 2-amino-4,6-diméthylpyridine par la 5-bromo-2,3-diamino-4,6-diméthylpyridine (préparation), on obtient le produit du titre. Solvant de recristallisation : acétone
Rendement : 49 %
Point de fusion : 202°C
Caractéristiques spectrales :
Résonance Magnétique Nucléaire ¹H (solvant CDCl₃)
CH₂ : singulet 2H ; 4.01 ppm

### EXEMPLE 8 : N-(4,6-DIMETHYLPYRIDIN-2-YL)-(5-BROMOTHIEN-2-YL)ACETAMIDE

En procédant comme dans l'exemple 1 mais en remplaçant l'acide (thién-2-yl)acétique par l'acide (5-bromo)(thién-2-yl)acétique, on obtient le produit du titre.
Point de fusion : 87 °C
Caractéristiques spectrales :
Résonance Magnétique Nucléaire ¹H (CDCl₃)
CH₂ : singulet 2 H : 3,83 ppm

### EXEMPLE 9: N-(4,6-DIMETHYLPYRIDIN-2-YL)-(THIEN-3-YL)ACETAMIDE

En procédant comme dans l'exemple 1 mais en remplaçant l'acide (thién-2-yl)acétique par l'acide (thién-3-yl)acétique, on obtient le produit du titre.
Recristallisation : éther isopropylique
Rendement : 65 %
Point de fusion : 123-124°C
Caractéristiques spectrales :
Infra-rouge
1660 cm⁻¹ : ν CO
Résonance Magnétique Nucléaire ¹H (CDCl₃)
CH₂-CO singulet 2H ; 3.74 ppm.

### EXEMPLE 10: N-(5-BROMO 4,6-DIMETHYLPYRIDIN-2-YL)-(THIEN-3-YL)ACETAMIDE

En procédant comme dans l'exemple 4 mais en remplaçant l'acide (thién-2-yl)acétique par l'acide (thién-3-yl)acétique, on obtient le produit du titre.
Recristallisation : acétone
Rendement : 55 %
Point de fusion : 211°C
Caractéristiques spectrales :
Infra-rouge
1650cm⁻¹ : ν CO
Résonance Magnétique Nucléaire ¹H (CDCl₃)
CH₂ CO singulet 2H ; 3.76 ppm.

### EXEMPLE 11: N-(5-BROMOPYRIDIN-2-YL)-(THIEN-3-YL)ACETAMIDE

En procédant comme dans l'exemple 9 mais en remplaçant la 2-amino 4,6-diméthyl pyridine par la 2-amino-5-bromopyridine, on obtient le produit du titre.
Solvant de recristallisation : éther isopropylique
Rendement : 50%
Point de fusion : 117°C
Caractéristiques spectrales :
Infra-rouge
1665 cm⁻¹ ; ν CO
Résonance Magnétique Nucléaire ¹H (solvant CDCl₃)
CH₂ : singulet 2H ; 3.72 ppm

### EXEMPLE 12: N-(3,5-DICHLOROPYRIDIN-2-YL)-(THIEN-3-YL)ACETAMIDE

En procédant comme dans l'exemple 9 mais en remplaçant la 2-amino-4,6-diméthylpyridine par la 2-amino-3,5-dichloropyridine, on obtient le produit du titre.
Recristallisation : éther isopropylique
Rendement: 30 %
Point de fusion : 159 - 160°C
Caractéristiques spectrales :
Infra-rouge
1680 cm⁻¹ : ν CO
Résonance Magnétique Nucléaire ¹H Solvant CDCl₃
CH₂: singulet ; 2H ; 3.97 ppm.

### EXEMPLE 13 : N-(3,5-DIBROMO-4,6-DIMETHYLPYRIDIN-2-YL)-(THIEN-3-YL) ACETAMIDE

En procédant comme dans l'exemple 9 mais en remplaçant la 2-amino-4,6-diméthylpyridine par la 2-amino-3,5-dibromo-4,6-diméthylpyridine, on obtient le produit du titre.
Point de fusion: 157°C

### EXEMPLE 14: N-(4,6-DIMETHYLPYRIDIN-2-YL)-(2-BROMO-THIEN-3-YL)ACETAMIDE

En procédant comme dans l'exemple 1 mais en remplaçant l'acide (5-bromothién-2-yl) acétique par l'acide (2-bromothién-3-yl)acétique, on obtient le produit du titre.
Point de fusion : 72°C
Caractéristiques spectrales:
Résonance Magnétique Nucléaire ¹H (CDCl₃)
CH₂: singulet ; 2H ; 3.70 ppm.

### EXEMPLE 15: N-(4,6-DIMETHYLPYRIDIN-2-YL)-(2,5-DIBROMO-THIEN-3-YL) ACETAMIDE

En procédant comme dans l'exemple 1 mais en remplaçant l'acide (thién-2-yl)acétique par l'acide (2,5-dibromothién-3-yl)acétique, on obtient le produit du titre.
Point de fusion: 109°C
Caractéristiques spectrales:
Résonance Magnétique Nucléaire ¹H (CDCl₃)
CH₂: singulet ; 2H ; 3.64 ppm

### EXEMPLE 16: N-ETHYL N-(4,6-DIMETHYLPYRIDIN-2-YL)-(THIEN-3-YL)ACETAMIDE

En procédant comme dans l'exemple 2 mais en remplaçant l'acide (thién-2-yl)acétique par l'acide (thién-3-yl)acétique, on obtient le produit du titre, huileux.
Rendement: 75 %
Indice de réfraction: 1,571
Caractéristiques spectrales:
Infra-rouge
1650 cm⁻¹ ν CO
Résonance Magnétique Nucléaire ¹H (solvant CDCl₃)
CH₃-CH₂ : triplet 3H ; δ :1.12 ppm
CH₃-CH₂ : quadruplet 2H ; δ : 3.33 ppm
CH₂-CO : singulet 2H ; δ : 3.55 ppm

### EXEMPLE 17: N-(2-AMINO-5-BROMO-4,6-DIMETHYLPYRIDIN-3-YL)-(THIEN-3-YL)ACETAMIDE

En procédant comme dans l'exemple 7 mais en remplaçant l'acide (thién-2-yl)acétique par l'acide (thién-3-yl)acétique, on obtient le produit du titre.
Recristallisation : acétone
Rendement : 50 %
Point de fusion : 194°C
Caractéristiques spectrales :
Infra-rouge
1635 cm⁻¹ ν CO
Résonance Magnétique Nucléaire ¹H (solvant CDCl₃)
CH₂ : singulet 2H ; 3.84 ppm.

### EXEMPLE 18: N-HEXYL-N(4,6-DIMETHYLPYRIDIN-2-YL)-(THIEN-3-YL)ACETAMIDE

En procédant comme dans l'exemple 3 mais en remplaçant l'acide (thién-2-yl)acétique par l'acide (thién-3-yl)acétique, on obtient le produit du titre.

### EXEMPLE 19: N-(4,6-DIMETHYLPYRIDIN-2-YL)-(2-CHLORO-BENZO[b]THIEN-3-YL)ACETAMIDE

En procédant comme dans l'exemple 1 mais en remplaçant l'acide (thién-2-yl)acétique par l'acide (2-chloro-benzo[b]thién-3-yl)acétique, on obtient le produit du titre.
Recristallisation : éther isopropylique
Rendement : 55 %
Point de fusion : 123 - 124°C
Caractéristiques spectrales :
Infra-rouge
1660 cm⁻¹ ν CO
Résonance Magnétique Nucléaire ¹H
CH₂ : singulet ; 2H ; 3.91 ppm
4-CH₃ : singulet ; 3H ; 2.22 ppm
6-CH₃ : singulet ; 3H ; 3.30 ppm.

### EXEMPLE 20 : N-(4,6-DIMETHYLPYRIDIN-2-YL)-(4-OXO[4H]-BENZOPYRAN-2-YL)CARBOXAMIDE

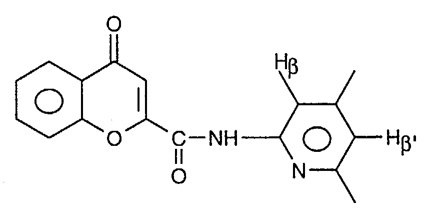

En procédant comme dans l'exemple 1 mais en remplaçant l'acide (thién-2-yl)acétique par l'acide 4-oxo[4H]-(benzopyran-2-yl)carboxylique, on obtient le produit du titre.
Recristrallisation : éther isopropylique
Rendement : 60 %
Point de fusion : 195°C
Caractéristiques spectrales :
Résonance Magnétique Nucléaire ¹H (solvant CDCl₃)
H_{β}' : singulet 1H ; 6.85 ppm
H_{β} : singulet 1H ; 8.02 ppm

### EXEMPLE 21: N-(4,6-DIMETHYLPYRIDIN-2-YL)-(PYRAZIN-2-YL)CARBOXAMIDE

Dissoudre 3 g d'acide (pyrazin-2-yl)carboxylique dans 20 ml de chlorure de thionyle. Laisser en contact 30 minutes à environ 60°C. Evaporer l'excès de chlorure de thionyle et laver plusieurs fois le chlorure d'acide obtenu.
Ce chlorure d'acide ainsi obtenu est repris dans 20 ml de dichloroéthane. Parallèlement dissoudre dans 30 ml de dichloroéthane, 3 g de 2-amino-4,6-diméthylpyridine. Ajouter 3 ml de triéthylamine puis la solution du chlorure d'acide précédemment préparé. Laisser en contact pendant 60 minutes. Filtrer puis évaporer le résidu. Chromatographier sur colonne de silice en éluant par du dichlorométhane.
Recristallisation : éther isopropylique
Rendement : 75 %
Point de fusion : 123 - 125°C
Caractéristiques spectrales :
Infra-rouge
1690 cm⁻¹ : ν CO
Résonance Magnétique Nucléaire ¹H (CDCl₃)
CH₃(4) : singulet 3H ; 2.37 ppm
CH₃(6) : singulet 3H ; 2.46 ppm.

### EXEMPLE 22: N-(5-BROMO-4,6-DIMETHYLPYRIDIN-2-YL)-(PYRAZIN-2-YL) CARBOXAMIDE

En procédant comme dans l'exemple 4 mais en remplaçant l'acide (thién-2-yl)acétique par l'acide (pyrazin-2-yl)carboxylique, on obtient le produit du titre.
Recristallisation : acétone
Rendement : 60 %
Point de fusion : 206°C
Caractéristques spectrales :
Infra-rouge
1680 cm⁻¹ : ν CO
Résonance Magnétique Nucléaire ¹H(CDCl₃)
H (pyridine) : singulet ; 8.17 ppm
H₆(pyrazine) : doublet dédoublé : 1H ; 8.62 ppm
H₅(pyrazine) : doublet: 1H ; 8.83 ppm, J5.6 ; 2.40 Hz

### EXEMPLE 23: N-(3,5-DIBROMO-4,6-DIMETHYLPYRIDIN-2-YL)-(PYRAZIN-2-YL) CARBOXAMIDE

En procédant comme dans l'exemple 5 mais en remplaçant l'acide (thién-2-yl)acétique par l'acide (pyrazin-2-yl)carboxylique, on obtient le composé du titre.
Point de fusion : 166°C

### EXEMPLE 24: N-(4,6-DIMETHYLPYRIDIN-2-YL)-(PYRAZIN-2-YL)THIOCARBOXAMIDE

Dissoudre 2,5 g de produit obtenu dans l'exemple 21 et 5,31 g de réactif de Lawesson dans 50 ml de toluène, porter au reflux pendant 4 h. Filtrer, évaporer le solvant. Purifier par chromatographie sur gel de silice en éluant au dichlorométhane.

Recueillir et recristalliser dans l'éther isopropylique
Rendement : 45 %
Point de fusion : 123°C
Caractéristiques spectrales :
Infra-rouge
1665 cm⁻¹ : ν C=N
Résonance Magnétique Nucléaire ¹H (solvant DMSOD₆)
CH₃(4) : singulet 3H ; 2.43 ppm
CH₃(6) : singulet 3H ; 2.52 ppm

### EXEMPLE 25: N-(4,6-DIMETHYLPYRIDIN-2-YL)-(PYRAZIN-2-YL)CARBOXAMIDE HYDRAZONE

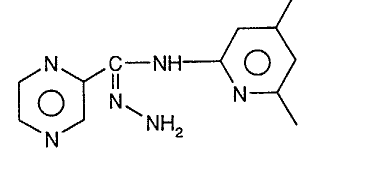

A 1 g de dérivé de l'exemple 24 en solution dans 30 ml d'éthanol, ajouter 0,6 ml de monohydrate d'hydrazine et laisser agir 30 minutes à température ambiante, sous agitation. Verser alors le milieu réactionnel dans de l'eau glacée. Agiter vigoureusement pendant 20 minutes. Filtrer, sécher et recristalliser le résidu dans l'éther isopropylique.
Rendement : 73 %
Point de fusion : 156°C
Caractéristiques spectrales :
Infra-rouge
3350 cm⁻¹ ν NH
Résonance Magnétique Nucléaire ¹H (CDCl₃)
CH₃(4) : singulet 3H ; 2.25 ppm
CH₃(6) : singulet 4H ; 2.40 ppm

### EXEMPLE 26: N-(4,6-DIMETHYLPYRIDIN-2-YL)-(PYRAZIN-2-YL)CARBAMIDOXIME

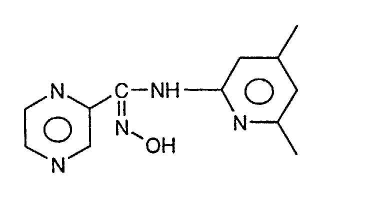

Dans un ballon, introduire 1, 65 g de dérivé obtenu dans l'exemple 24 et 45 ml d'éthanol. Dissoudre à chaud, ajouter 2,34 g de chlorhydrate d'hydroxylamine puis 1, 79 g de carbonate de sodium dissous dans 20 ml d'eau. Porter aux reflux 30 minutes. Diluer le milieu réactionnel à l'eau, filtrer, sécher. Recueillir le produit et recristalliser dans un mélange méthanol/chloroforme.
Rendement : 80 %
Point de fusion : 191°C
Caractéristiques spectrales :
Infra-rouge
3270 cm⁻¹ ν (HN)
2500 - 2900 ν (OH)
Résonance Magnétique Nucléaire ¹H (DMSOD₆)
CH₃(4) : singulet 3H ; 1.80 ppm
CH₃(6) : singulet 3H ; 2.17 ppm

### EXEMPLE 27: N-(4,6-DIMETHYLPYRIDIN-2-YL)-O-METHYL-(PYRAZIN-2-YL) CARBAMIDOXIME

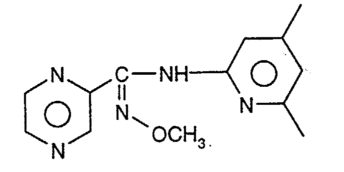

En procédant comme dans l'exemple 26 mais en remplaçant le chlorhydrate d'hydroxylamine par le chlorhydrate de méthoxylamine, on obtient le produit du titre.
Recristallisation : méthanol
Rendement : 88 %
Point de fusion : 133°C
Caractéristiques spectrales :
Infra-rouge
1610 - 1560 cm⁻¹ : ν CN
Résonance Magnétique Nucléaire ¹H (CDCl₃)
CH₃(4) : singulet 3H ; 2.10 ppm
CH₃(6) : singulet 3H ; 2.15 ppm
OCH₃ : singulet 3H ; 4.02 ppm

### EXEMPLE 28: N-(4,6-DIMETHYLPYRIDIN-2-YL)-O-BENZYL-(PYRAZIN-2-YL) CARBAMIDOXIME

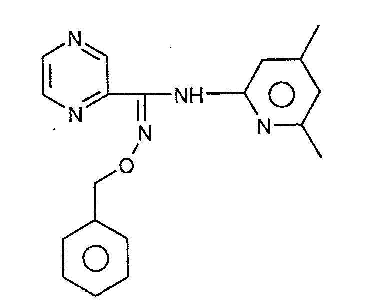

En procédant comme dans l'exemple 26 mais en remplaçant le chlorhydrate d'hydroxylamine par le chlorhydrate de benzyloxylamine, on obtient le produit du titre.
Point de fusion : 84°C

### EXEMPLE 29 : N-METHYL-N'-(4,6-DIMETHYLPYRIDIN-2-YL)-(PYRAZIN-2-YL) CARBOXAMIDINE

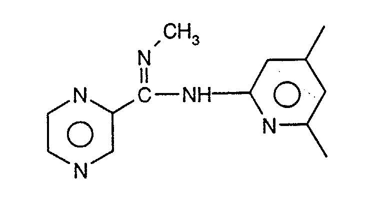

Dans un ballon contenant de l'éthanol, ajouter 1,23 g de dérivé obtenu dans l'exemple 24 et 1,95 g d'une solution aqueuse à 40 % de méthylamine. Laisser agir à température ambiante; filtrer et évaporer. Cristalliser l'huile obtenue dans l'éther diéthylique et recristalliser dans l'éther isopropylique.
Rendement : 64 %
Point de fusion : 103 -104°C
Caractéristiques spectrales :
Infra-rouge
1625, 1610 cm⁻¹
Résonance Magnétique Nucléaire 1H (solvant DMSO D₆)
CH₃(4) : singulet 3H ; 2.03 ppm
CH₃(6) : singulet 3H ; 2.18 ppm
N-CH₃: singulet 3H ; 2.91 ppm

### EXEMPLE 30: N-(4,6-DIMETHYLPYRIDIN-2-YL)-PYRAZIN-2-YL-CARBOXAMIDINE

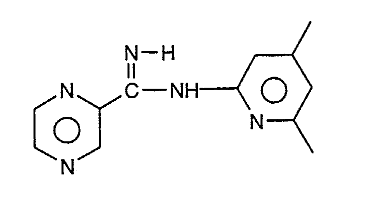

Dans un ballon dicol, 1,2 g (4,91 mmol) de produit obtenu dans l'exemple 24 et 40 ml d'éthanol sont introduits. Faire barboter un courant d'ammoniac. Filtrer l'insoluble puis évaporer. Recristalliser le résidu dans l'éther isopropylique.
Rendement: 95 %
Point de fusion : 148°C
Caractéristiques spectrales :
Infra-rouge
ν CN 1625, 1600 cm⁻¹
Résonance Magnétique Nucléaire ¹H (CDCl₃)
CH₃(4) singulet 3H : 2.31 ppm
CH₃(6) singulet 3H : 2.49 ppm

### EXEMPLE 31: N-(2-AMINO-5-BROMO-4,6-DIMETHYLPYRIDIN-3-YL)-(PYRAZIN-2-YL) CARBOXAMIDE

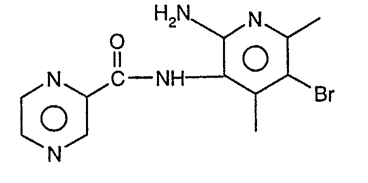

En procédant comme dans l'exemple 7 mais en remplaçant l'acide (thién-2-yl) acétique par l'acide (pyrazin-2-yl)carboxylique, on obtient le produit du titre.
Rendement : 52 %
Point de fusion : 224°C
Caractéristiques spectrales :
Résonance Magnétique Nucléaire ¹H (CDCl₃)
CH₃(4) : singulet(3H) ; 2.35 ppm
CH₃(6) : singulet (3H) ; 2.56 ppm

### EXEMPLE 32: N-OXYDE DU N-(4,6-DIMETHYLPYRIDIN-2-YL)-PYRAZIN-2-YL-CARBOXAMIDE

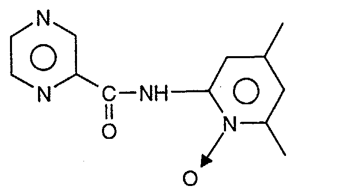

Ajouter sous agitation à 1 g de composé obtenu dans l'exemple 21 une solution de 10 ml d'acide acétique glacial et 0,7 ml d'eau oxygénée (35 %). Chauffer le milieu réactionnel à 70°C pendant 7 h puis concentrer sous pression réduite et à basse température.
Refroidir la solution résiduelle. Filtrer le solide blanc obtenu. Laver à l'eau glacée, sécher, purifier par chromatographie sur gel de silice. Recristalliser le résidu dans un mélange chlorure de méthylène / éther isopropylique.
Rendement : 66 %
Point de fusion : 210°C
Caractéristiques spectrales :
Infra-rouge
1225 cm⁻¹ : ν NO
Résonance Magnétique Nucléaire ¹H (CDCl₃)
H₆ : doublet dédoublé 1H H₆

### EXEMPLE 33: N-OXYDE DU N-(4,6-DIMETHYLPYRIDIN-2-YL)-(THIEN-2-YL)ACETAMIDE

En procédant comme dans l'exemple 32 mais en remplaçant le N-(4,6-diméthyl pyridin-2-yl)-(pyrazin-2-yl)carboxamide (obtenu à l'exemple 21) par le N-(4,6-diméthyl pyridin-2-yl)-(thién-2-yl)acétamide (obtenu à l'exemple 1), on obtient le produit du titre.
Point de fusion : 154-155°C

### EXEMPLE 34: N-OXYDE DU N-(4,6-DIMETHYLPYRIDIN-2-YL)-(THIEN-3-YL)ACETAMIDE

En procédant comme dans l'exemple 32 mais en utilisant le composé obtenu à l'exemple 9, on obtient le produit du titre.
Point de fusion : 154°C

### EXEMPLES 35 A 41 :

En procédant comme dans les exemples 24 à 30 mais en utilisant au départ le N-(4,6-diméthylpyridin-2-yl)-(thién-2-yl)acétamide, on obtient respectivement

### EXEMPLE 35: N-(4,6-DIMETHYLPYRIDIN-2-YL)-(THIEN-2-YL)THIOACETAMIDE

Point de fusion : 64°C

### EXEMPLE 36: N-(4,6-DIMETHYLPYRIDIN-2-YL)-(THIEN-2-YL)ACETAMIDE HYDRAZONE

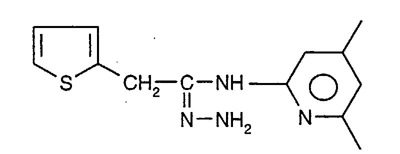

### EXEMPLE 37: N-(4,6-DIMETHYLPYRIDIN-2-YL)-(THIEN-2-YL)ACETAMIDOXIME

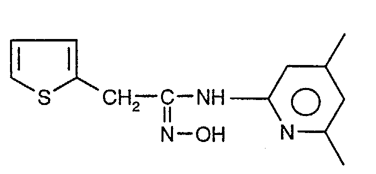

### EXEMPLE 38: N-(4,6-DIMETHYLPYRIDIN-2-YL)-O-METHYL-(THIEN-2-YL) ACETAMIDOXIME

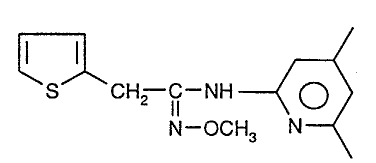

### EXEMPLE 39: N-(4,6-DIMETHYLPYRIDIN-2-YL)-O-BENZYL-(THIEN-2-YL) ACETAMIDOXIME

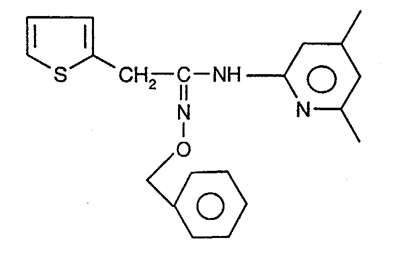

Point de fusion : 87°C

### EXEMPLE 40: N-METHYL-N'-(4,6-DIMETHYLPYRIDIN-2-YL)-(THIEN-2-YL)ACETAMIDINE

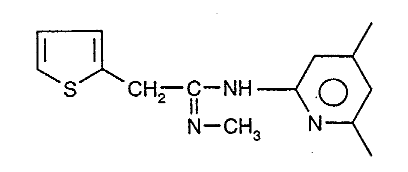

### EXEMPLE 41: N-(4,6-DIMETHYLPYRIDIN-2-YL)-(THIEN-2-YL)ACETAMIDINE

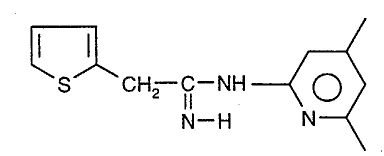

### EXEMPLES 42 A 48 :

En procédant comme dans les exemples 24 à 30 mais en utilisant au départ le N-(4,6-diméthylpyridin-2-yl)-(thién-3-yl)acétamide on obtient respectivement :

### EXEMPLE 42: N-(4,6-DIMETHYLPYRIDIN-2-YL)-(THIEN-3-YL)THIOACETAMIDE

Point de fusion : 69°C

### EXEMPLE 43: N-(4,6-DIMETHYLPYRIDIN-2-YL)-(THIEN-3-YL)ACETAMIDE HYDRAZONE

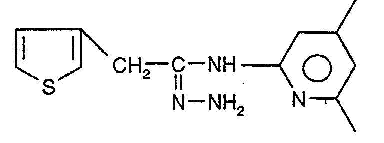

### EXEMPLE 44: N-(9,6-DIMETHYLPYRIDIN-2-YL)-(THIEN-3-YL)ACETAMIDOXIME

Point de fusion : 131°C

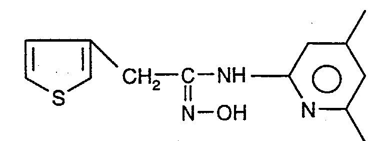

### EXEMPLE 45: N-(4,6-DIMETHYLPYRIDIN-2-YL)-(THIEN-3-YL)O-METHYL ACETAMIDOXIME

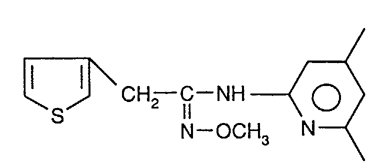

### EXEMPLE 46: N-(4,6-DIMETHYLPYRIDIN-2-YL)-(THIEN-3-YL)O-BENZYL ACETAMIDOXIME

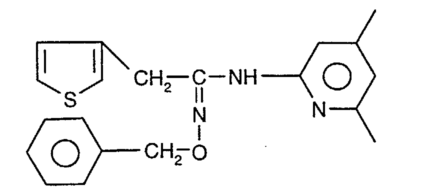

### EXEMPLE 47: N-METHYL-N'-(4,6-DIMETHYLPYRIDIN-2-YL)-(THIEN-3-YL) ACETAMIDINE

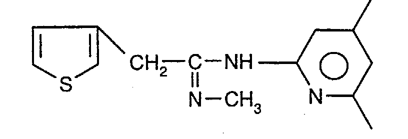

### EXEMPLE 48: N-(4,6-DIMETHYLPYRIDIN-2-YL)-(THIEN-3-YL)ACETAMIDINE

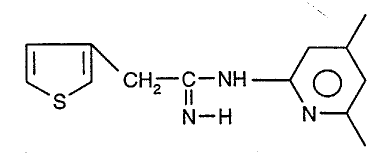

### EXEMPLE 49: N-(4,6-DIMETHYL-5-NITRO PYRIDIN-2-YL)-(PYRAZIN-2-YL) CARBOXAMIDE

En procédant comme dans l'exemple 22 mais en utilisant au départ la 2-amino-5-nitro-4,6-diméthylpyridine, on obtient le produit du titre.
Point de fusion : 158°C

### EXEMPLE 50: N-(4,6-DIMETHYL-PYRIDIN-2-YL)-PHTALIMIDO-ACETAMIDE

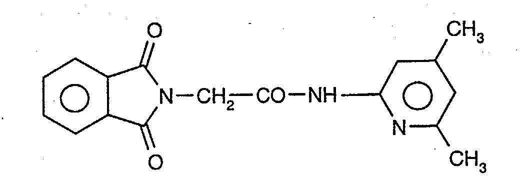

En procédant comme dans l'exemple 1 mais en remplaçant l'acide (thién-2-yl)acétique par l'acide (phtalamido) acétique, on obtient le produit du titre.
Point de fusion : 212-213°C
Caractéristiques spectrales :
Résonance Magnétique Nucléaire ¹H (Solvant CDCl₃)
CH₂ : singulet ; 2H : 4,54 ppm

### EXEMPLE 51: N-(4,6-DIMETHYL PYRIDIN-2-YL)-(PYRIDIN-4-YL)CARBOXAMIDE

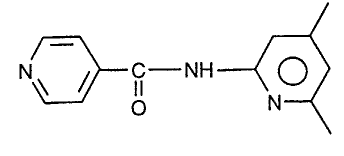

En procédant comme dans l'exemple 1 mais en remplaçant l'acide (thién-2-yl) par l'acide (pyridin-4-yl)carboxylique, on obtient le produit du titre.
Point de fusion : 142°C

### EXEMPLE 52: N-(4,6-DIMETHYL PYRIDIN-2-YL)-(2-METHYLTHIO-PYRIDIN-3-YL)ACETAMIDE

En procédant comme dans l'exemple 1 mais en remplaçant l'acide (thién-2-yl)acétique par l'acide (2-méthylthio-pyridin-3-yl)carboxylique, on obtient le produit du titre.
Point de fusion : 164°C

### EXEMPLE 53: N-(4,6-DIMETHYL PYRIDIN-2-YL)-(2-HYDROXY-PYRIDIN-3-YL) CARBOXAMIDE

En procédant comme dans l'exemple 1 mais en remplaçant l'acide (thién-2-yl)acétique par l'acide (2-hydroxy-pyridin-3-yl)carboxylique, on obtient le produit du titre.
Point de fusion : 245°C

### EXEMPLE 54: N-(4,6-DIMETHYL PYRIDIN-2-YL)-(2-CHLORO-PYRIDIN-3-YL) CARBOXAMIDE

En procédant comme dans l'exemple 1 mais en remplaçant l'acide (thién-2-yl)acétique par l'acide (2-chloro-pyridin-3-yl)carboxylique, on obtient le produit du titre.
Point de fusion : 91°C

### EXEMPLE 55: N-(4,6-DIMETHYL PYRIDIN-2-YL)-(2-CHLOROBENZO[b]THIEN-3-YL) CARBOXAMIDE

En procédant comme dans l'exemple 1 mais en remplaçant l'acide thién-2-yl)acétique par l'acide (2-chlorobenzo[b]thién-3-yl)carboxylique, on obtient le produit du titre.

### EXEMPLES 56 à 62 :

En procédant comme dans les exemples 24 à 30 mais en utilisant au départ le N-(4,6-diméthyl pyridin-2-yl)-(2-chlorobenzo[b]thién-3-yl)acétamide (composé de l'exemple 19), on obtient respectivement:

### EXEMPLE 56: N-(4,6-DIMETHYL PYRIDIN-2-YL)-(2-CHLOROBENZO[b]THIEN-3-YL)THIOACETAMIDE

### EXEMPLE 57: N-(4,6-DIMETHYL PYRIDIN-2-YL)-(2-CHLOROBENZO[b]THIEN-3-YL)ACETAMIDE HYDRAZONE

### EXEMPLE 58: N-(4,6-DIMETHYL PYRIDIN-2-YL)-(2-CHLOROBENZO[b]THIEN-3-YL)ACETAMIDOXIME

### EXEMPLE 59: N-(4,6-DIMETHYL PYRIDIN-2-YL)-O-METHYL-(2-CHLOROBENZO[b] THIEN-3-YL)ACETAMIDOXIME

### EXEMPLE 60: N-(4,6-DIMETHYL PYRIDIN-2-YL)-O-BENZYL-(2-CHLOROBENZO[b] THIEN-3-YL)ACETAMIDOXIME

### EXEMPLE 61: N'-METHYL-N-(4,6-DIMETHYL PYRIDIN-2-YL)-(2-CHLOROBENZO[b] THIEN-3-YL)ACETAMIDINE

### EXEMPLE 62: N-(4,6-DIMETHYL PYRIDIN-2-YL)-(2-CHLOROBENZO[b]THIEN-3-YL)ACETAMIDINE

### EXEMPLE 63: N-(4,6-DIMETHYL PYRIDIN-2-YL)-4-OXO[4H]BENZOPYRAN-2-YL ACETAMIDE

En procédant comme dans l'exemple 1 mais en remplaçant l'acide (thién-2-yl)acétique par l'acide (4-oxo[4H]benzopyran-2-yl)acétique, on obtient le produit du titre.

### EXEMPLES 64 A 70 :

En procédant comme dans les exemples 24 à 30 mais en utilisant au départ le N-(4,6-diméthylpyridin-2-yl)-(4-oxo[4H]bentopyran-2-yl)carboxamide (composé de l'exemple 20), on obtient respectivement :

### EXEMPLE 64: N-(4,6-DIMETHYL PYRIDIN-2-YL)-(4-OXO[4H]BENZOPYRAN-2-YL)THIOCARBOXAMIDE

### EXEMPLE 65: N-(4,6-DIMETHYL PYRIDIN-2-YL)-(4-OXO[4H]BENZOPYRAN-2-YL)CARBOXAMIDE HYDRAZONE

### EXEMPLE 66: N-(4,6-DIMETHYL PYRIDIN-2-YL)-(4-OXO[4H]BENZOPYRAN-2-YL)CARBAMIDOXIME

### EXEMPLE 67: N-(4,6-DIMETHYL PYRIDIN-2-YL)-O-METHYL-(4-OXO[4H]BENZOPYRAN-2-YL)CARBAMIDOXIME

### EXEMPLE 68: N-(4,6-DIMETHYL PYRIDIN-2-YL)-O-BENZYL-(4-OXO[4H]BENZOPYRAN-2-YL)CARBAMIDOXIME

### EXEMPLE 69: N-(4,6-DIMETHYL PYRIDIN-2-YL-N'-METHYL-(4-OXO[4H]BENZOPYRAN -2-YL)CARBOXAMIDINE

### EXEMPLE 70: N-(4,6-DIMETHYL PYRIDIN-2-YL)-(4-OXO[4H]BENZOPYRAN-2-YL) CARBOXAMIDINE

### EXEMPLES 71 A 77 :

En procédant comme dans les exemples 24 à 30 mais en utilisant au départ le N-(4,6-diméthylpyridin-2-yl)-(pyridin-4-yl)carboxamide (composé de l'exemple 51), on obtient respectivement:

### EXEMPLE 71: N-(4,6-DIMETHYL PYRIDIN-2-YL)-(PYRIDIN-4-YL)THIOCARBOXAMIDE

### EXEMPLE 72: N-(4,6-DIMETHYL PYRIDIN-2-YL)-(PYRIDIN-4-YL)CARBOXAMIDE HYDRAZONE

### EXEMPLE 73: N-(4,6-DIMETHYL PYRIDIN-2-YL)-(PYRIDIN-4-YL)CARBAMIDOXIME

### EXEMPLE 74: N-(4,6-DIMETHYL PYRIDIN-2-YL)-(PYRIDIN-4-YL)-O-METHYL CARBAMIDOXIME

### EXEMPLE 75: N-(4,6-DIMETHYL PYRIDIN-2-YL)-(PYRIDIN-4-YL)-O-BENZYL CARBAMIDOXIME

### EXEMPLE 76: N-METHYL-N'-(4,6-DIMETHYL PYRIDIN-2-YL)-(PYRIDIN-4-YL)CARBOXAMIDINE

### EXEMPLE 77: N-(4,6-DIMETHYL PYRIDIN-2-YL)-(PYRIDIN-4-YL)CARBOXAMIDINE

### EXEMPLE 78: N-(4,6-DIMETHYL PYRIDIN-2-YL)-(PYRIDIN-4-YL)ACETAMIDE

En procédant comme dans l'exemple 1 mais en remplaçant l'acide (thién-2-yl)acétique par l'acide (pyridin-4-yl)acétique, on obtient le produit du titre.

### EXEMPLE 79: N-(4,6-DIMETHYL PYRIDIN-2-YL)-(THIEN-3-YL)PROPIONAMIDE

En procédant comme dans l'exemple 1 mais en utilisant au départ l'acide (thién-3-yl)propionique, on obtient le produit du titre.

### EXEMPLE 80: N-(4,6-DIMETHYL PYRIDIN-2-YL)-(THIEN-3-YL)BUTANAMIDE

En procédant comme dans l'exemple 1 mais en utilisant au départ l'acide (thién-3-yl)butanoïque, on obtient le produit du titre.

### EXEMPLE 81: N-(4,6-DIMETHYL PYRIDIN-2-YL)-(THIEN-3-YL)PROPENAMIDE

En procédant comme dans l'exemple 1 mais en utilisant au départ l'acide (thién-3-yl)propènoïque, on obtient le produit du titre.

### EXEMPLE 82: N-(4,6-DIMETHYL PYRIDIN-2-YL)-(BENZOPYRAN-3-YL)CARBOXAMIDE

En procédant comme dans l'exemple 1 mais en utilisant au départ l'acide (benzopyran-3-yl)carboxylique, on obtient le produit du titre.

### EXEMPLE 83: N-(4,6-DIMETHYL PYRIDIN-2-YL)-(BENZOPYRAN-3-ZYL)ACETAMIDE

En procédant comme dans l'exemple 1 mais en utilisant au départ l'acide (benzopyran-3-yl)acétique, on obtient le produit du titre.

### EXEMPLE 84: N-(4,6-DIMETHYL PYRIDIN-2-YL)-(CHROMAN-3-YL)CARBOXAMIDE

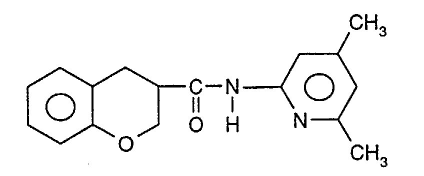

En procédant comme dans l'exemple 1 mais en utilisant au départ l'acide (chroman-3-yl)carboxylique, on obtient le produit du titre.

### EXEMPLE 85: N-(4,6-DIMETHYL PYRIDIN-2-YL)-(CHROMAN-3-YL)ACETAMIDE

En procédant comme dans l'exemple 1 mais en utilisant au départ l'acide chroman-3-ylacétique, on obtient le produit du titre.

### EXEMPLE 86: N-(4,6-DIMETHYLPYRIDIN-2-YL)-(5-METHYLPYRAZIN-2-YL)CARBOXAMIDE

En procédant comme dans l'exemple 2 mais en remplaçant l'acide (thién-2-yl)acétique par l'acide (5-méthylpyrazin-2-yl)carboxylique et la 2-éthylamino-4,6-diméthylpyridine par la 2-amino 4,6-diméthylpyridine, on obtient le produit du titre sous forme d'une poudre blanche que l'on recristallise dans un mélange acétate d'éthyle / chloroforme (7/3).
Point de fusion : 169°C

### EXEMPLES 87 A 93 :

En procédant comme dans les exemples 24 à 30 mais en utilisant au départ le N-(4,6-diméthylpyridin-2-yl)-(5-méthylpyrazin-2-yl)carboxamide (composé de l'exemple 86), on obtient respectivement :

### EXEMPLE 87: N-(4,6-DIMETHYLPYRIDIN-2-YL)-(5-METHYLPYRAZIN-2-YL) THIOCARBOXAMIDE

Recristallisation : éther isopropylique / éther de pétrole (9/1)
Rendement : 43 %
Point de fusion : 129°C
Caractéristiques spectrales :
Infra-rouge
3260 cm⁻¹ : ν NH
Résonance Magnétique Nucléaire ¹H (CDCl₃)
CH₃(5 de la pyrazine) : singulet, 3H, 2,67 ppm

### EXEMPLE 88: N-(4,6-DIMETHYL PYRIDIN-2-YL)-(5-METHYLPYRAZIN-2-YL)CARBOXAMIDE HYDRAZONE

### EXEMPLE 89: N-(4,6-DIMETHYL PYRIDIN-2-YL)-(5-METHYLPYRAZIN-2-YL) CARBOXAMIDOXIME

Point de fusion : 173°C

### EXEMPLE 90: N-(4,6-DIMETHYL PYRIDIN-2-YL)-(5-METHYLPYRAZIN-2-YL) O-METHYL CARBOXAMIDOXIME

Point de fusion : 137°C

### EXEMPLE 91: N-(4,6-DIMETHYL PYRIDIN-2-YL)-(5-METHYLPYRAZIN-2-YL) O-BENZYL CARBOXAMIDOXIME

### EXEMPLE 92: N-METHYL-N'-(4,6-DIMETHYL PYRIDIN-2-YL)-(5-METHYLPYRAZIN-2-YL)CARBOXAMIDINE

### EXEMPLE 93: N-(4,6-DIMETHYL PYRIDIN-2-YL)-(5-METHYL-PYRAZIN-2-YL)CARBOXAMIDINE

### EXEMPLE 94: N-(4,6-DIMETHYLPYRIDIN-2-YL)-3-(PYRROLIDIN-1-YL)-BUT-2-ENAMIDE

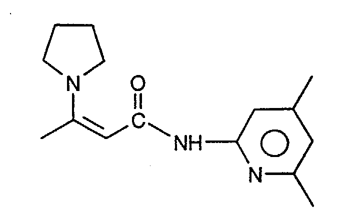

Dans un ballon de 250 ml surmonté d'un Dean Stark, introduire 4.52 g (23.6 mmol) de N-(4,6 diméthylpyridin-2-yl)-2-acétoacétamide, obtenu par condensation d'une pyridine et d'acétylacétate d'éthyle, 2.02 g (28.6 mmol) de pyrrolidine et 55 ml de benzène. Chauffer au reflux pendant une heure. Evaporer le solvant. Laver les cristaux par l'éther isopropylique. Recueillir le produit du titre, de couleur écru.
point de fusion : 132°C

### EXEMPLE 95: N-(4,6-DIMETHYLPYRIDIN-2-YL)-3-(PYRROLIDIN-1-YL)BUTANAMIDE

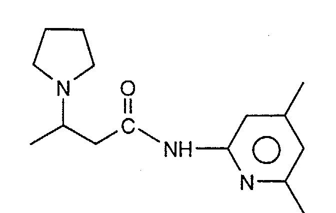

Dans un ballon de 250 ml, inroduire 1.37 g (5.3 mmol) du composé obtenu à l'exemple 94 et 60 ml de méthanol. Y ajouter lentement 1 g (27 mmol) de borhydrure de sodium. Laisser sous agitation à température ambiante pendant 1h30. Evaporer le solvant, reprendre par de l'eau. Extraire par dichlorométhane. Evaporer, purifier le produit brut par passage sur colonne de gel de silice en éluant par un mélange dichlorométhane/éthanol (90/10). Le produit obtenu est visqueux et cristallise lentement.

### EXEMPLE 96: N-(4,6-DIMETHYL PYRIDIN-2-YL)-(3-ETHOXYCARBONYL-5-METHYL-ISOXAZOL-4-YL)CARBOXAMIDE

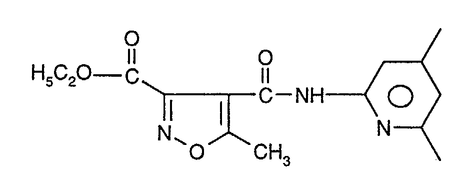

Refroidir à 0°C une solution de 3.5 g (13.51 mmol) du composé obtenu à l'exemple 94 dans 30 ml de dichlorométhane. Y ajouter en une seule fois 2.62 g de chlorooximidoacétate d'éthyle. Poursuivre l'agitation à 0°C pendant 3 heures. Verser le milieu réactionnel dans de l'eau. Extraire par du dichlorométhane. Laver la phase organique par une solution à 5% de chlorure d'hydrogène puis par une solution saturée de bicarbonate de sodium. Sécher et évaporer le solvant. Purifier par passage sur colonne de gel de silice en éluant par de l'éther isopropylique. Le produit obtenu est sous forme de cristaux blancs.
Point de fusion : 108°C
Caractéristiques spectrales :
Résonnance Magnétique Nucléaire ¹H(CDCl₃) :
CH₂ : quadruplet 2H : 4,60 ppm

### EXEMPLE 97: N-(4,6-DIMETHYL PYRIDIN-2-YL)-(3-CARBOXY-5-METHYL-ISOXAZOL-4YL)CARBOXAMIDE

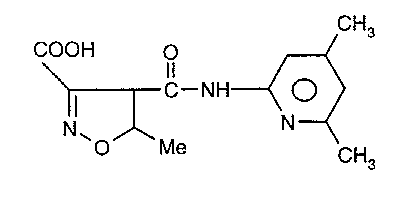

Refroidir à -15°C une solution de 0.27 g (11.5mmol) d'hydroxyde de lithium dans 3 ml d'eau et 15 ml de méthanol. Y ajouter lentement et sous agitation 1.5 g (5.19 mmol) du produit obtenu à l'exemple 96. Poursuivre l'agitation pendant une heure. Acidifier par une solution de chlorure d'hydrogène diluée. Laisser la température remonter à 0°C et agiter pendant 30 min. Filtrer, recueillir le produit du titre sous forme de poudre blanche. Laver par de l'oxyde de t-butylméthyle.
Point de fusion : 183°C

### EXEMPLE 98: N-(4,6-DIMETHYL PYRIDIN-2-YL)-3-(PIPERIDIN-1-YL)BUT-2-ENAMIDE

En procédant comme dans l'exemple 94 mais en remplaçant la pyrrolidine par la pipéridine, on obtient le produit du titre.

### EXEMPLE 99: N-(4,6-DIMETHYL PYRIDIN-2-YL)-3-(PYRROLIN-1-YL)BUT-2-ENAMIDE

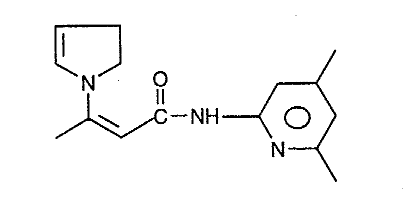

En procédant comme dans l'exemple 94 mais en remplaçant la pyrrolidine par la pyrroline, on obtient le produit du titre.

### EXEMPLE 100: N-(4,6-DIMETHYLPYRIDIN-2-YL)-(1-METHYL-PYRROL-2-YL)ACETAMIDE

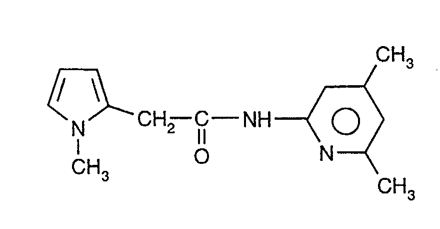

En procédant comme dans l'exemple 1 mais en remplaçant l'acide (thién-2-yl)acétique par l'acide (1 -méthyl-pyrrol-2-yl)acétique on obtient le produit du titre.
Point de fusion : 85°C

### ETUDE PHARMACOLOGIQUE DES DERIVES DE L'INVENTION

### EXEMPLE A : ETUDE DE LA TOXICITE AIGUE

La toxicité aigüe a été appréciée après administration orale à des lots de 8 souris (26 ± 2 grammes) d'une dose de 650 mg.kg⁻¹. Les animaux ont été observés à intervalles réguliers au cours de la première journée et quotidiennement pendant les 2 semaines suivant le traitement.
Il apparaît que les composés de l'invention sont totalement atoxiques. Aucun décès n'est observé après administration d'une dose de 650 mg.kg⁻¹. On ne constate pas de troubles après administration de cette dose.

### EXEMPLE B : ETUDE DE L'ACTIVITE ANTIINFLAMMATOIRE

La méthode retenue est celle de l'oedème plantaire à la carraghénine. Le protocole utilisé est le suivant : on administre dans la plante du pied droit de rats Sprague-Dawley de poids moyen 250 g la carraghénine à 1 % dans 0,2 ml d'une solution saline à 9 %. Une heure et deux heures plus tard, on mesure le volume de la patte par pléthysmographie.

Les composés de l'invention sont administrés à 10 mg/kg par voie orale 30 minutes avant l'administration de carraghénine. Une solution saline est injectée dans la plante du pied gauche qui sert de témoin.

Les composés de l'invention permettent de réduire l'augmentation de volume du pied droit par rapport à celui du pied gauche, de manière hautement significative (Table 1).

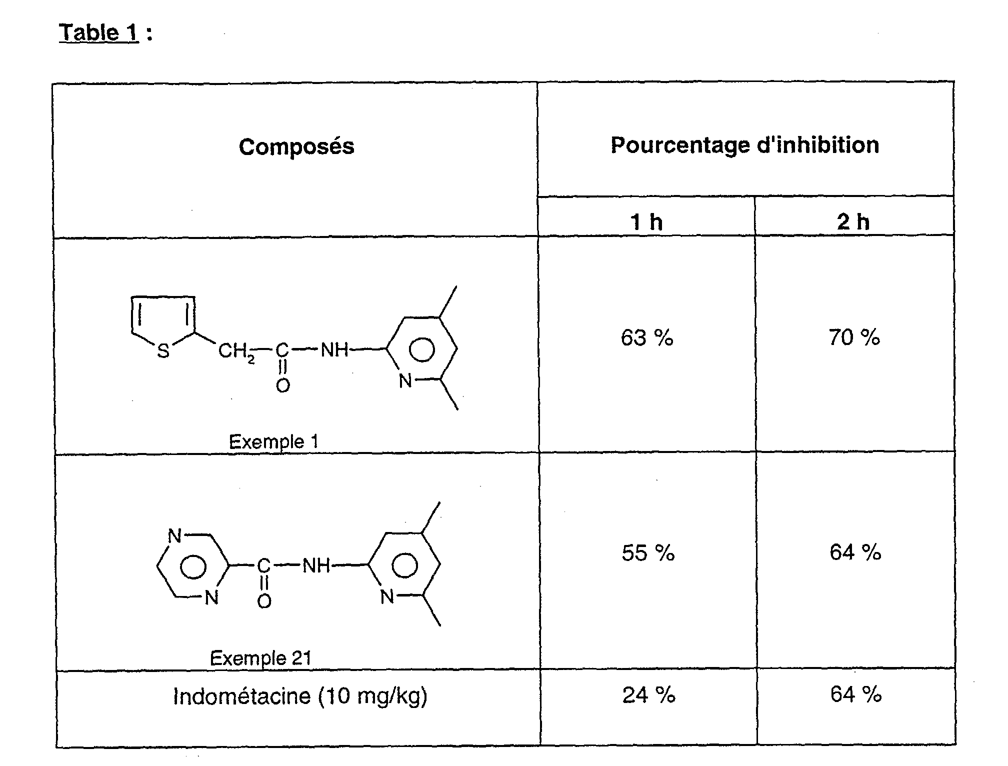

Les composés de l'invention inhibent très fortement l'inflammation induite par la carraghénine dès 1 h et sont plus actifs que l'indométacine, pris comme référence et administrée dans les mêmes conditions.

### EXEMPLE C : ETUDE DE L'ACTIVITE DIURETIQUE

On utilise des groupes de 3 rats à jeun. Chaque groupe reçoit 25 ml/kg par voie orale (p.o) d'eau distillée administrée avec les produits de l'invention à une dose de 3 mg/kg.

Le volume urinaire est mesuré pendant les 6 heures suivant l'administration.

Ainsi par exemple le composé de l'exemple 25 permet d'augmenter le volume urinaire d'un facteur 2,8 par rapport au rat non traité.

Administré à une dose de 5 mg/kg p.o, le furosémide, pris comme référence, permet d'augmenter le volume urinaire d'un facteur 3.

Le pouvoir diurétique des produits de l'invention est donc comparable à celui du furosémide.

### EXEMPLE D : MISE EN EVIDENCE DE L'ACTIVITE ANTI-INFLAMMATOIRE CUTANEE (TOPIQUE) EN AIGU

L'ester de phorbol (Phorbol 12-myristate 13-acetate) (5 µg) est appliqué en topique sur les surfaces antérieures et postérieures de l'oreille droite de la souris 30 minutes après application du véhicule (éthanol 95 %) ou de l'agent (1 mg). La différence d'épaisseur entre l'oreille droite et l'oreille gauche (oedème) est mesurée 6 heures après application.

Le pourcentage d'inhibition de l'inflammation cutanée par rappport à un groupe d'animaux traité à l'éthanol 95 % en topique est calculé. Le composé de l'exemple 1, à 1 mg/oreille, permet une diminution de l'inflammation de 63 %. Pris comme référence, I'indométacine, à une dose de 2,5 mg/oreille, permet une diminution de l'inflammation de 67 %.

### EXEMPLE E : ACTIVITE EN CURATIF ET APRES APPLICATION TOPIQUE REPETEE DANS LE MODELE ETABLI D'INFLAMMATION CHRONIQUE DE L'OREILLE SOUMISE A UNE APPLICATION REPETEE D'ESTER DE PHORBOL (PMA) PENDANT 15 JOURS (MODELE "PERTINENT" DU PSORIASIS)

L'ester de phorbol (1 µg) est appliqué en topique sur les surfaces antérieures et postérieures de l'oreille droite de la souris aux jours 0, 2, 4, 7, 9, 11 et 14. Le véhicule ou l'agent est appliqué en topique 2 fois par jour, aux jours 7, 8, 9, 10, 11, 12, 13 et 14, et une seule fois le 15ème jour. Les jours 7, 9, 11 et 14, le véhicule ou l'agent est appliqué 30 minutes avant et après l'ester de phorbol.

Deux paramètres sont mesurés:
1. Différence d'épaisseur entre les oreilles droite et gauche tous les jours et les jours 0, 2, 4, 7, 9, 11 et 14, 6 heures après application répétée de l'ester de phorbol.
   Après application répétée d'ester de phorbol et après 15 jours, I'épaisseur traduit non seulement la présence d'un oedème et d'une infiltration de cellules type neutrophiles monocytes-macrophages et lymphocytes dans le tissu cutané (inflammation) mais aussi une augmentation de l'épaisseur de l'épiderme (hyperplasie épidermique secondaire à une prolifération de kératinocytes). Ces deux processus constituent les bases physiopathologiques du psoriasis.
2. Différence de poids ("ear punches") entre les oreilles droite et gauche au 10ème jour. Dans ce modèle chronique, 15 jours après application répétée d'ester de phorbol, les agents antiinflammatoires classiques inhibiteurs de la cycloxygénase (indométhacine, piroxicam) sont inactifs en topique mais par contre des médicaments actuellement utilisés dans le traitement du psoriasis (cyclosporine A, corticoïdes) sont actifs en topique. Aussi, la cyclosporine A et l'hydrocortisone ont été utilisées comme subtances de référence. Un groupe recevant de l'éthanol sert de témoin.
   Les produits de l'invention en topique, curatif et réitéré, inhibent entre les 8ème et 10ème jours l'inflammation chronique de l'oreille droite (mesurée d'une part, par la différence d'épaisseur entre l'oreille droite et l'oreille gauche d'autre part, par la différence de poids entre les deux oreilles après sacrifice de l'animal au 15ème jour) induite par une application répétée d'ester de phorbol chez la souris.
   Par exemple, le composé de l'exemple 17, à une dose de 0,5 mg/oreille, permet de diminuer la différence d'épaisseur entre les deux oreilles de 60 %, et permet de diminuer la différence de poids de 38 %.

### EXEMPLE F : RECHERCHE D'UNE ACTIVITE DE TYPE ANTIARTHRITIQUE CHEZ LE RAT

On utilise des groupes de 5 rats Lewis male ou femelle d'un poids de 130 à 150 g. Une suspension de 0,3 mg de Mycobactérium tuberculosis tués dans 0,1 cm³ d'huile minérale (Adjuvant de Freund complet, CFA) est administrée dans la région subplantaire de la patte arrière droite le Jour 1. Les volumes des pattes arrières sont mesurés par déplacement d'eau les Jours 0, 1, 5, 14 et 18. Les rats sont pesés aux jours 0 et 18. Les produits à tester sont placés en suspension dans de la carboxyméthyl cellulose et administrés oralement pendant 5 jours consécutifs, des jours 1 à 5.
Parallèlement, un groupe témoin est utilisé afin d'éliminer les artefacts résultant de la manipulation des animaux. Un groupe traité par un produit de référence (hydrocortisone) permet la validation du test.

Au Jour 18, le composé de l'exemple 1 permet ainsi une diminution de volume de la patte arrière droite de 47 %.

Les produits de l'invention possèdent une puissante action inhibitrice dans ce modèle ce qui les situent comme de très intéressants candidats pour le traitement de l'arthrite.

### COMPOSITIONS PHARMACEUTIQUES

### EXEMPLE A:

### COMPRIMES POUR LE TRAITEMENT DES MALADIES INFLAMMATOIRES ET MALADIES RENALES

### Composés dosés à 10 mg de N-(4,6-diméthylpyridin-2-yl)-(thién-3-yl)acétamide

| Formule de préparation pour 1000 comprimés | |
|---|---|
| N-(4,6-diméthylpyridin-2-yl)-(thién-3-yl)acétamide | 10 g |
| Amidon de blé | 35 g |
| Amidon de maïs | 65 g |
| Lactose | 65 g |
| Stéarate de Magnésium | 2 g |
| Silice | 1 g |
| Hydroxypropylcellulose | 2 g |

### EXEMPLE B:

### POMMADE DESTINEE AU TRAITEMENT DU PSORIASIS ET DOSEE A 1 % DE N-(4,6-DIMETHYLPYRIDIN-2-YL)-(THIEN-2-YL)ACETAMIDE

Formule de préparation pour 100 kg
N-(4,6-diméthylpyridin-2-yl)-(thién-2-yl)acétamide 1000 g
Excipient en quantité suffisante pour 100 kg
(Alcools cétylique, stéarylique, isopropylique ; lanoline, monostéréate de polyéthylène glycol,
eau distillée de laurier cerise).

## Revendications

1. Dérivés de formule générale (I): 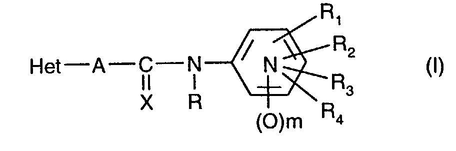 dans laquelle
m vaut 0 ou 1,
le symbole 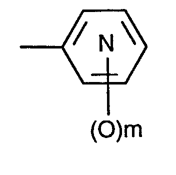 représentant lorsque m vaut 0, le noyau pyridine, et, lorsque m vaut 1, le N-oxyde de la pyridine, le système pyridinique 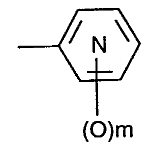 étant fixé au groupement 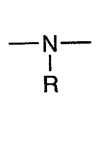 qui le porte soit en position 2, soit en position 3 de la pyridine ;
R₁, R₂, identiques ou différents, sont choisis, chacun indépendamment l'un de l'autre, parmi hydrogène, amino, alkylamino, dialkylamino, alkyle, hydroxy, alkoxy, nitro et halogène,
R₃, R₄, identiques ou différents sont choisis chacun indépendamment l'un de l'autre parmi amino, alkylamino, dialkylamino, alkyle, hydroxy, alkoxy, nitro et halogène,
R représente un atome d'hydrogène ou un groupement alkyle,
A représente une liaison simple ; et dans ce cas Het représente un groupement choisi parmi pyrazine, pyrazine substituée (à l'exception du N-(4,6-diméthylpyridin-2-yl)-(5-méthylpyrazin-2-yl)carboxamide), benzothiophène, benzothiophène substitué, 4-oxo[4H] benzopyrane, 4-oxo[4H]benzopyrane substitué, pyrrole, pyrrole substitué, pyrroline, pyrroline substituée, pyrrolidine, pyrrolidine substituée, pipéridine, pipéridine substituée, pyridine, pyridine substituée, benzopyrane, benzopyrane substitué par un ou plusieurs groupements alkyle, chromane, chromane substitué par un ou plusieurs groupements alkyle, 3-carboxy-5-alkylisoxazole, 3-alkoxycarbonyl-5-alkyl-isoxazole, phtalimido et phtalimido substitué,
ou bien A représente un groupement alkylène non substitué ou substitué par un ou plusieurs groupements alkyle, ou un groupement alcénylène non substitué ou substitué par un ou plusieurs groupements alkyle ; et dans ce cas Het représente un groupement choisi parmi thiophène, thiophène substitué, pyrazine, pyrazine substituée, benzothiophène, benzothiophène substitué, 4-oxo[4H]benzopyrane, 4-oxo[4H]benzopyrane substitué, pyrrole, pyrrole substitué, pyrroline, pyrroline substituée, pyrrolidine, pyrrolidine substituée, pipéridine, pipéridine substituée, pyridine, pyridine substituée, benzopyrane, benzopyrane substitué par un ou plusieurs groupements alkyle, chromane, chromane substitué par un ou plusieurs groupements alkyle, 3-carboxy-5-alkyl-isoxazole, 3-alkoxycarbonyl-5-alkyl-isoxazole, phtalimido et phtalimido substitué,
X représente un atome d'oxygène, un atome de soufre, un groupement imino ou un groupement imino substitué par un groupement choisi parmi alkyle, alkoxy, hydroxy, amino, arylalkyloxy et aryloxy,
leurs énantiomères et diastéréoisomères et leurs sels d'addition à un acide ou une base pharmaceutiquement acceptable,
étant entendu que, sauf précisions contraires :
- le terme "substitué" affectant les systèmes thiophène, pyrazine, benzothiophène, 4-oxo[4H]benzopyrane, pyrrole, pyrroline, pyrrolidine, pipéridine, pyridine et phtalimido, signifie que ces systèmes sont substitués par un ou plusieurs groupements choisis parmi alkyle, alkoxy, trifluorométhyle, hydroxy, halogène, thiol et alkylthio,
- les termes "alkyle" et "alkoxy", "alkylène", désignent des groupements linéaires ou ramifiés comprenant de 1 à 6 atomes de carbone,
- le terme "aryle" désigne un radical phényle ou naphthyle,
- le terme "alcénylène" désigne une chaîne insaturée linéaire ou ramifiée contenant de 2 à 6 atomes de carbone.

2. Composés selon la revendication 1 ayant la formule (IA): 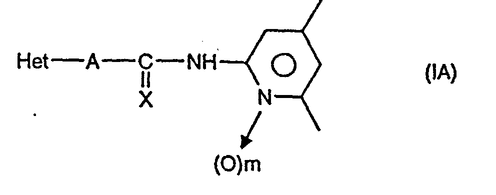 dans laquelle m représente 0 ou 1, et
- soit Het représente le groupement thiophène et A représente un méthylène,
- soit Het représente le groupement pyrazine ou le groupement pyrazine substitué par un groupement alkyle et A est une liaison simple,
et X représente un atome d'oxygène un atome de soufre, un groupement imino ou un groupement imino substitué par un groupement hydroxy, un groupement méthoxy, un groupement méthyle, un groupement amino, ou un groupement benzyloxy,
leurs énantiomères et diastéréoisomères et leurs sels d'addition à un acide pharmaceutiquement acceptable.

3. Composé selon la revendication 1 qui est le N-(4,6-diméthyl-pyridin-2-yl)-(thién-2-yl)acétamide, son N-oxyde et ses sels d'addition à un acide pharmaceutiquement acceptable.

4. Composé selon la revendication 1 qui est le N-(4,6-diméthyl-pyridin-2-yl)-(thién-3-yl) acétamide, son N-oxyde et ses sels d'addition à un acide pharmaceutiquement acceptable.

5. Composé selon la revendication 1 qui est la N-(4,6-diméthyl-pyridin-2-yl)-(pyrazin-2-yl)carbamidoxime, de formule : 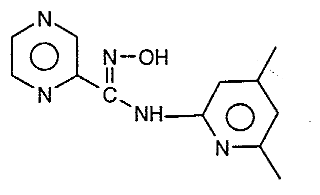 son N-oxyde et ses sels d'addition à un acide pharmaceutiquement acceptable.

6. Composé selon la revendication 1 qui est la N-(4,6-diméthyl-pyridin-2-yl)-O-méthyl(pyrazin-2-yl)carbamidoxime, de formule : 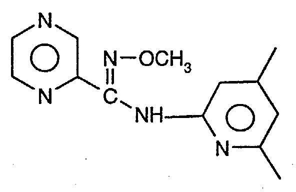 son N-oxyde, ses énantiomères et ses sels d'addition à un acide pharmaceutiquement acceptable.

7. Composé selon la revendication 1 qui est le N-(4,6-diméthyl-pyridin-2-yl)-(5-méthylpyrazin-2-yl)thiocarboxamide, son N-oxyde et ses sels d'addition à un acide pharmaceutiquement acceptable.

8. Composé selon la revendication 1 qui est le N-(4,6-diméthyl-pyridin-2-yl)-(pyrazin-2-yl)carboxamidine, son N-oxyde, et ses sels d'addition à un acide pharmaceutiquement acceptable.

9. Composé selon la revendication 1 qui est le N-méthyl-N'-(4,6-diméthyl-pyridin-2-yl)-(2-pyrazin-2-yl)carboxamidine, son N-oxyde, et ses sels d'addition à un acide pharmaceutiquement acceptable.

10. Procédé de préparation des composés de formule (I) selon la revendication 1 caractérisé en ce que l'on utilise comme matière première un dérivé de formule (1) : 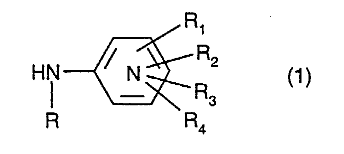 où R, R₁, R₂, R₃, R₄ et le cycle 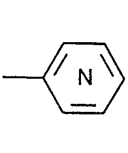 ont la même définition que dans la revendication 1,
• soit que l'on condense avec un dérivé de formule (2) :
Het-A-COOH (2)
où Het et A ont la même définition que dans la revendication 1,
pour conduire à un dérivé de formule (I/a) : 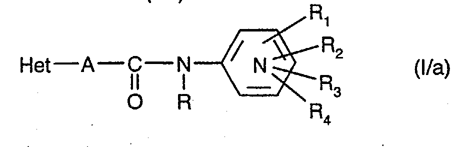 où Het, A, R, R₁, R₂, R₃ et R₄ et le cycle 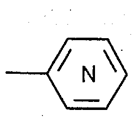 ont la même définition que précédemment, dérivé de formule (I/a), qui est soumis à un agent de thionation pour conduire à un dérivé de formule (I/b) : 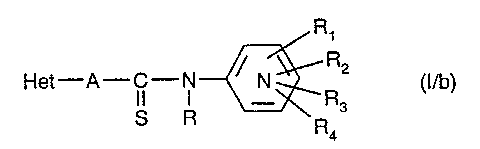 où Het, A, R, R₁, R₂, R₃ et R₄ et le cycle 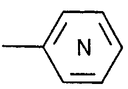 ont la même signification que précédemment, dérivé de formule (I/b), qui est condensé avec un dérivé de formule (3) : 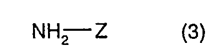 où Z représente un atome d'hydrogène, un groupement hydroxy, alkyle, alkoxy, amino, arylalkyloxy et aryloxy pour conduire à un dérivé de formule (I/c): 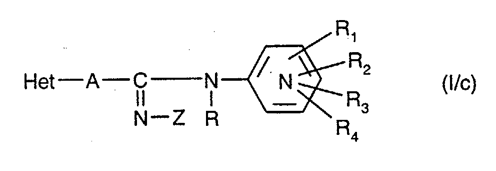 où Het, A, R, R₁, R₂, R₃,R₄, Z et le cycle 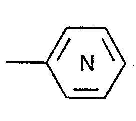 ont la même définition que précédemment,
• soit que l'on fait réagir avec un composé de formule (4) : 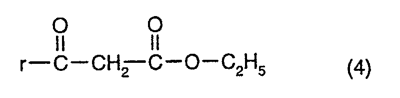 où r est un groupement alkyle,
afin d'obtenir des composés de formule (5) : 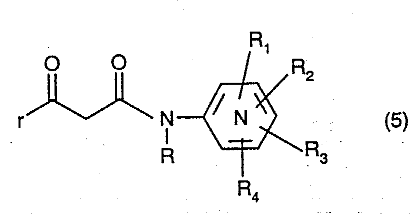 dans laquelle r, R, R₁, R₂, R₃, R₄ sont tels que définis précédemment, qui sont mis à réagir avec un composé de formule Het-H où Het est tel que défini dans la formule (I),
afin d'obtenir des composés de formule (I/d), 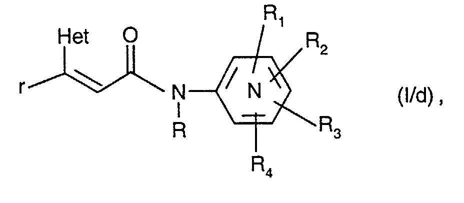 dans laquelle Het, r, R, R₁, R₂, R₃, R₄ sont tels que définis précédemment,
composés de formule (I/d) qui peuvent, si on le désire, être réduits, par l'action de borohydrure de sodium par exemple, pour donner des composés de formule (I/e) : 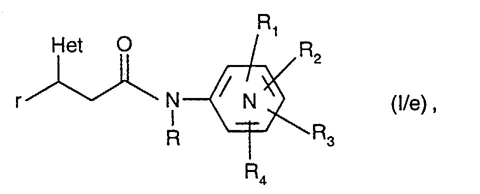 dans laquelle Het, r, R, R₁, R₂, R₃, R₄ sont tels que définis précédemment,
ou bien qui peuvent, dans le cas où Het représente un groupement pyrrolidine, réagir avec du chlorooximidoacétate d'alkyle de formule (6) : 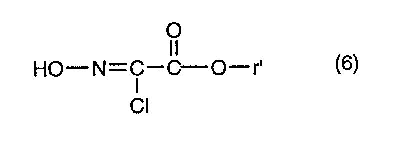 où r' est un groupement alkyle,
pour donner des composés de formule (I/f) : 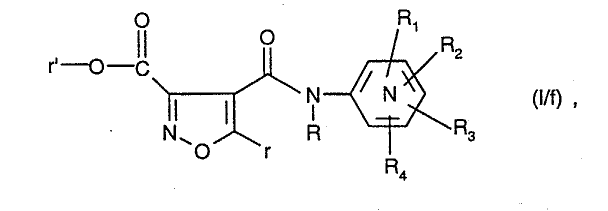 dans laquelle r, r', R, R₁, R₂, R₃, R₄ sont tels que définis précédemment,
composés de formule (I/f) qui peuvent réagir, si on le désire, avec de l'hydroxyde de lithium, afin d'obtenir des composés de formule (I/g) : 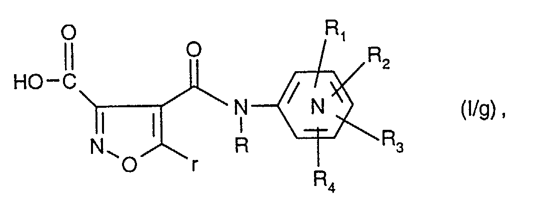 dans laquelle r, R, R₁, R₂, R₃, R₄ sont tels que définis précédemment,
dérivés de formule (I/a), (I/b), (I/c), (I/d), (I/e), (I/f), (I/g) que l'on peut transformer, si on le désire, en dérivés N-oxydes de la pyridine par action d'eau oxygénée,
les dérivés de formule (I/a), (I/b), (I/c), (I/d), (I/e), (I/f), (I/g) ainsi que leurs N-oxydes formant l'ensemble des dérivés de formule (I) selon la revendication 1, dérivés de formule (I) dont on peut séparer les énantiomères et diastéréoisomères et que l'on peut salifier par un acide ou une base pharmaceutiquement acceptable.

11. Composition pharmaceutique contenant comme principe actif au moins un composé selon l'une des revendications 1 à 9 en combinaison avec un ou plusieurs excipients ou véhicules inertes, non toxiques, pharmaceutiquement acceptables.

12. Composition pharmaceutique selon la revendication 11 contenant au moins un principe actif selon l'une des revendications 1 à 9 utilisable dans le traitement des troubles inflammatoires, des maladies inflammatoires rénales, de l'arthrite, du psoriasis et de l'eczéma.

## Patentansprüche

1. Derivate der allgemeinen Formel (I): 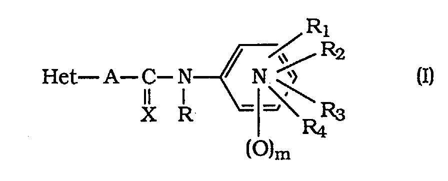 in der
m 0 oder 1, das Symbol 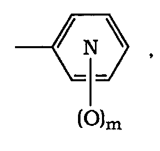 wenn m 0 bedeutet, den Pyridinkern und wenn m 1 bedeutet, das N-Oxid des Pyridins, wobei das Pyridinsystem 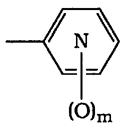 an der es tragenden Gruppe 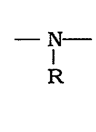 in der 2-Stellung oder in der 3-Stellung des Pyridins gebunden ist;
R₁ und R₂, die gleichartig oder verschieden sein können, jeweils unabhängig voneinander aus Wasserstoff, Amino, Alkylamino, Dialkylamino, Alkyl, Hydroxy, Alkoxy, Nitro und Halogen ausgewählt sind,
R₃ und R₄, die gleichartig oder verschieden sein können,jeweils unabhängig voneinander aus Amino, Alkylamino, Dialkylamino, Alkyl, Hydroxy, Alkoxy, Nitro und Halogen ausgewählt sind,
R ein Wasserstoffatom oder eine Alkylgruppe,
A eine Einfachbindung; und in diesem Fall Het eine Gruppe ausgewählt aus Pyrazin, substituiertem Pyrazin (mit Ausnahme von N-(4,6-Dimethylpyridin-2-yl)-(5-methylpyrazin-2-yl)-carboxamid), Benzothiophen, substituiertem Benzothiophen, 4-Oxo-[4H]-benzopyran, substituiertem 4-Oxo-[4H]-benzopyran, Pyrrol, substituiertem Pyrrol, Pyrrolin, substituiertem Pyrrolin, Pyrrolidin, substituiertem Pyrrolidin, Piperidin, substituiertem Piperidin, Pyridin, substituiertem Pyridin, Benzopyran, durch eine oder mehrere Alkylgruppen substituiertem Benzopyran, Chroman, durch eine oder mehrere Alkylgruppen substituiertem Chroman, 3-Carboxy-5-alkyl-isoxazol, 3-Alkoxycarbonyl-5-alkyl-isoxazol, Phthalimido und substituiertem Phthalimido ausgewählte Gruppe darstellt,
oder A eine Alkylengruppe, die nicht substituiert oder durch eine oder mehrere Alkylgruppen substituiert ist, eine Alkenylengruppe, die nicht substituiert oder durch eine oder mehrere Alkylgruppen substituiert ist; wobei in diesem Fall Het eine aus Thiophen, substituiertem Thiophen, Pyrazin, substituiertem Pyrazin, Benzothiophen, substituiertem Benzothiophen, 4-Oxo-[4H]-benzopyran, substituiertem 4-Oxo-[4H]-benzopyran, Pyrrol, substituiertem Pyrrol, Pyrrolin, substituiertem Pyrrolin, Pyrrolidin, substituiertem Pyrrolidin, Piperidin, substituiertem Piperidin, Pyridin, substituiertem Pyridin, Benzopyran, durch eine oder mehrere Alkylgruppen substituiertem Benzopyran, Chroman, durch eine oder merhere Alkylgruppen substituiertem Chroman, 3-Carboxy-5-alkyl-isoxazol, 3-Alkoxycarbonyl-5-alkyl-isoxazol, Phthalimido und substituiertem Phthalimido ausgewählte Gruppe darstellt und
X ein Sauerstoffatom, ein Schwefelatom, eine Iminogruppe oder eine durch eine aus Alkyl, Alkoxy, Hydroxy, Amino, Arylalkoxy und Aryloxy ausgewählte Gruppe substituierte Iminogruppe bedeuten,
deren Enantiomere und Diastereoisomere und deren Additionssalze mit einer pharmazeutisch annehmbaren Base, oder pharmazeutisch annehmbaren Säure, wobei, wenn nichts Genaueres angegeben ist:
- der bezüglich der Thiophen-, Pyrazin-, Benzothiophen-, 4-Oxo-[4H]-benzopyran-, Pyrrol-, Pyrrolin-, Pyrrolidin-, Piperidin-, Pyridin- und Phthalimido-Systeme verwendete Begriff "substituiert" bedeutet, daß diese Systeme durch eine oder mehrere Gruppen ausgewählt aus Alkyl, Alkoxy, Trifluormethyl, Hydroxy, Halogen. Thiol und Alkylthio substituiert sind,
- die Begriffe "Alkyl", "Alkoxy" und "Alkylen" für geradkettige oder verzweigte Gruppen mit 1 bis 6 Kohlenstoffatomen stehen,
- der Begriff "Aryl" für eine Phenyl- oder Naphthylgruppe steht und
- der Begriff "Alkenylen" für eine geradkettige oder verzweigte ungesättigte Kette mit 2 bis 6 Kohlenstoffatomen steht.

2. Verbindungen nach Anspruch 1 der Formel (IA): 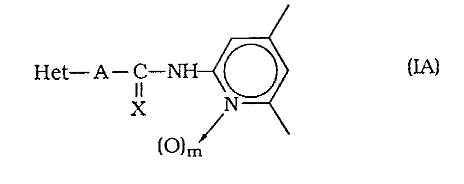 in der m 0 oder 1 und
- entweder Het die Thiophengruppe und A eine Methylengruppe,
- oder Het die Pyrazingruppe oder die durch eine Alkylgruppe substituierte Pyrazingruppe und A eine Einfachbindung
und X ein Sauerstoffatom, ein Schwefelatom, eine Iminogruppe oder eine Iminogruppe, die durch eine Hydroxylgruppe, eine Methoxygruppe, eine Methylgruppe, eine Aminogruppe oder eine Benzyloxygruppe substituiert ist, bedeuten, deren Enantiomere und Diastereoisomere und deren Additionssalze mit einer pharmazeutisch annehmbaren Säure.

3. Verbindung nach Anspruch 1, nämlich N-(4,6-Dimethyl-pyridin-2-yl)-(thien-2-yl)-acetamid, dessen N-Oxid und dessen Additionssalze mit einer pharmazeutisch annehmbaren Säure.

4. Verbindung nach Anspruch 1, nämlich N-(4,6-Dimethyl-pyridin-2-yl)-(thien-3-yl)-acetamid, dessen N-Oxid und dessen Additionssalze mit einer pharmazeutisch annehmbaren Säure.

5. Verbindung nach Anspruch 1, nämlich N-(4,6-Dimethyl-pyridin-2-yl) -(pyrazin-2-yl)-carbamidoxim der Formel: 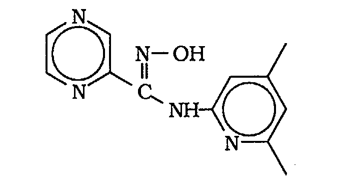 dessen N-Oxid und dessen Additionssalze mit einer pharmazeutisch annehmbaren Säure.

6. Verbindung nach Anspruch 1, nämlich N-(4,6-Dimethyl-pyridin-2-yl)-O-methyl-(pyrazin-2-yl)-carbamidoxim der Formel: 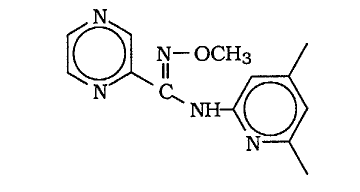 dessen N-Oxid, dessen Enantiomere und dessen Additionssalze mit einer pharmazeutisch annehmbaren Säure.

7. Verbindung nach Anspruch 1, nämlich N-(4,6-Dimethyl-pyridin-2-yl)-(5-methylpyrazin-2-yl)-thiocarboxamid, dessen N-Oxid und dessen Additionssalze mit einer pharmazeutisch annehmbaren Säure.

8. Verbindung nach Anspruch 1, nämlich N-(4,6-Dimethyl-pyridin-2-yl)-(pyrazin-2-yl)-carboxamidin, dessen N-Oxid und dessen Additionssalze mit einer pharmazeutisch annehmbaren Säure.

9. Verbindung nach Anspruch 1, nämlich N-Methyl-N'-(4,6-dimethyl-pyridin-2-yl)-(2-pyrazin-2-yl)-carboxamidin, dessen N-Oxid und dessen Additionssalze mit einer pharmazeutisch annehmbaren Säure.

10. Verfahren zur Herstellung der Verbindungen der Formel (I) nach Anspruch 1, dadurch gekennzeichnet, daß man als Ausgangsmaterial ein Derivat der Formel (1) verwendet: 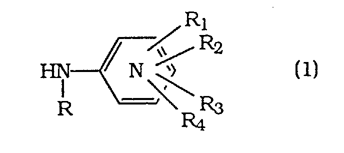 in der R, R₁, R₂, R₃, R₄ und der Ring 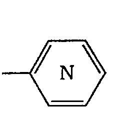 die in Anspruch 1 angegebenen Bedeutungen besitzen, verwendet,
• welches man entweder mit einem Derivat der Formel (2):
Het - A - COOH (2)
in der Het und A die in Anspruch 1 angegebenen Bedeutungen besitzen, kondensiert zur Bildung eines Derivats der Formel (I/a): 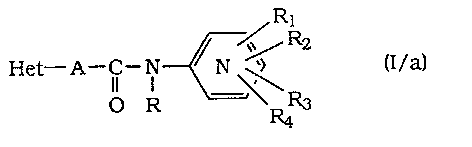 in der Het, A, R, R₁, R₂, R₃ und R₄ und der Ring 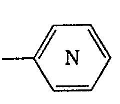 die oben angegebenen Bedeutungen besitzen,
welches Derivat der Formel (I/a) man der Einwirkung eines Thionierungsmittels unterwirft zur Bildung eines Derivats der Formel (I/b): 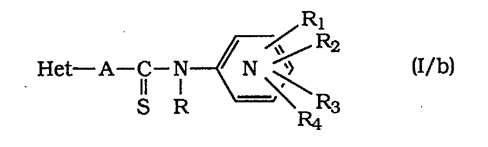 in der Het, A, R, R₁, R₂, R₃ und R₄ und der Ring 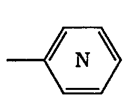 die oben angegebenen Bedeutungen besitzen,
welches Derivat der Formel (I/b) mit einem Derivat der Formel (3):
NH₂ - Z (3)
in der Z ein Wasserstoffatom, eine Hydroxylgruppe, eine Alkylgruppe, eine Alkoxygruppe, eine Aminogruppe, eine Arylalkyloxygruppe oder eine Aryloxygruppe bedeutet, kondensiert zur Bildung eines Derivats der Formel (I/c): 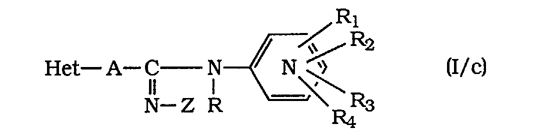 in der Het, A, R, R₁, R₂, R₃, R₄ und Z und der Ring 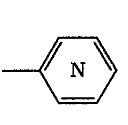 die oben angegebenen Bedeutungen besitzen,
• oder mit einer Verbindung der Formel (4): 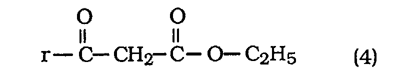 in der r eine Alkylgruppe darstellt, umsetzt,
zur Bildung der Verbindungen der Formel (5): 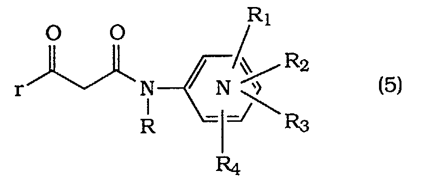 in der r, R, R₁, R₂, R₃ und R₄ die oben angegebenen Bedeutungen besitzen, welche man mit einer Verbindung der Formel Het-H, in der Het die bezüglich der Formel (I) angegebenen Bedeutungen besitzt, umsetzt,
zur Bildung der Verbindungen der Formel (I/d): 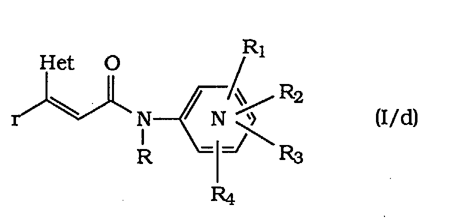 in der Het, r, R, R₁, R₂ R₃ und R₄ die oben angegebenen Bedeutungen besitzen, welche Verbindungen der Formel (I/d) gewünschtenfalls reduziert werden können, beispielsweise mit Natriumborhydrid, zur Bildung der Verbindungen der Formel (I/e): 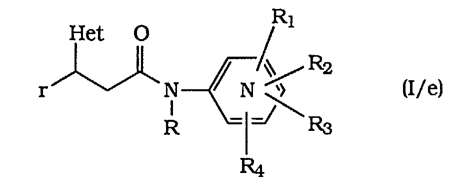 in der Het, r, R, R₁, R₂, R₃ und R₄ die oben angegebenen Bedeutungen besitzen, oder welche dann, wenn Het eine Pyrrolidingruppe darstellt, mit Chloroximidoessigsäurealkylester der Formel (6): 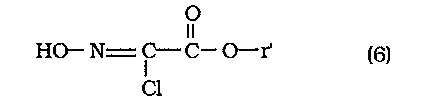 in der r' eine Alkylgruppe darstellt, umgesetzt werden können,
zur Bildung der Verbindungen der Formel (I/f): 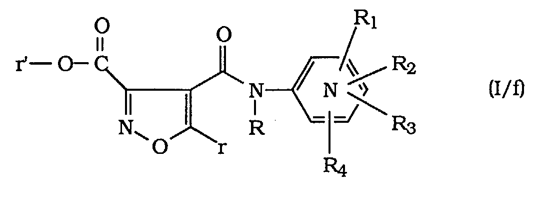 in der r, r', R, R₁, R₂, R₃ und R₄ die oben angegebenen Bedeutungen besitzen, welche Verbindungen der Formel (I/f) man gewünschtenfalls mit Lithiumhydroxid umsetzen kann zur Bildung der Verbindungen der Formel (I/g): 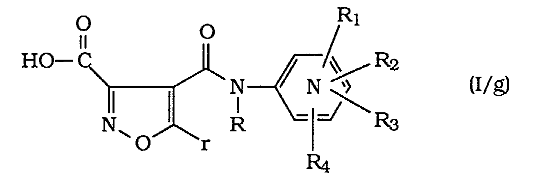 in der r, R, R₁, R₂, R₃ und R₄ die oben angegebenen Bedeutungen besitzen,
welche Derivate der Formeln (I/a), (I/b), (I/c), (I/d), (I/e), (I/f) und (I/g) man gewünschtenfalls durch Einwirkung von Wasserstoffperoxid in die N-Oxide des Pyridins umwandeln kann,
welche Derivate der Formeln (I/a), (I/b), (I/c), (I/d), (I/e), (I/f) und (I/g) sowie deren N-Oxide gemeinsam die Derivate der Formel (I) nach Anspruch 1 bilden, welche Derivate der Formel (I) man in die Enantiomeren und Diastereoisomeren auftrennen und mit einer pharmazeutisch annehmbaren Säure oder Base in ihre Salze überführen kann.

11. Pharmazeutische Zubereitung enthaltend als Wirkstoff mindestens eine Verbindung nach einem der Ansprüche 1 bis 9 in Kombination mit einem oder mehreren inerten, nichttoxischen, pharmazeutisch annehmbaren Trägermaterialien oder Bindemitteln.

12. Pharmazeutische Zubereitung nach Anspruch 11 enthaltend mindestens einen Wirkstoff nach einem der Ansprüche 1 bis 9 zur Verwendung bei der Behandlung von Entzündungszuständen, Entzündungserkrankungen der Nieren, Arthritis, Psoriasis und Ekzemen.

## Claims

1. Compounds of the general formula (I): 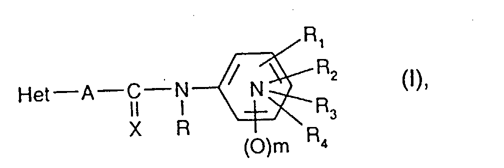 in which
m is 0 or 1,
the symbol 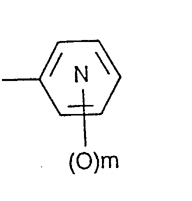 representing the pyridine ring when m is 0 and the N-oxide of pyridine when m is 1, the pyridine system 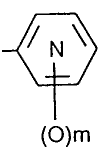 being bonded to the group 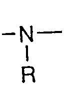 which carries it either in the 2-position or in the 3-position of the pyridine;
R₁ and R₂, which are the same or different, are selected, each independently of the other, from hydrogen, amino, alkylamino, dialkylamino, alkyl, hydroxy, alkoxy, nitro and halogen,
R₃ and R₄, which are the same or different, are selected, each independently of the other, from amino, alkylamino, dialkylamino, alkyl, hydroxy, alkoxy, nitro and halogen,
R represents a hydrogen atom or an alkyl group,
A represents a single bond; and, in that case, Het represents a group selected from pyrazine, substituted pyrazine (with the exception of N-(4,6-dimethylpyridin-2-yl)-(5-methylpyrazin-2-yl)carboxamide), benzothiophene, substituted benzothiophene, 4-oxo[4H]benzopyran, substituted 4-oxo[4H]benzopyran, pyrrole, substituted pyrrole, pyrroline, substituted pyrroline, pyrrolidine, substituted pyrrolidine, piperidine, substituted piperidine, pyridine, substituted pyridine, benzopyran, benzopyran substituted by one or more alkyl groups, chroman, chroman substituted by one or more alkyl groups, 3-carboxy-5-alkyl-isoxazole, 3-alkoxycarbonyl-5-alkyl-isoxazole, phthalimido and subtituted phthalimido,
or A represents an alkylene group that is unsubstituted or substituted by one or more alkyl groups, or represents an alkenylene group that is unsubstituted or substituted by one or more alkyl groups; and, in that case, Het represents a group selected from thiophene, substituted thiophene, pyrazine, substituted pyrazine, benzothiophene, substituted benzothiophene, 4-oxo[4H]benzopyran, substituted 4-oxo[4H]benzopyran, pyrrole, substituted pyrrole, pyrroline, substituted pyrroline, pyrrolidine, substituted pyrrolidine, piperidine, substituted piperidine, pyridine, substituted pyridine, benzopyran, benzopyran substituted by one or more alkyl groups, chroman, chroman substituted by one or more alkyl groups, 3-carboxy-5-alkyl-isoxazole, 3-alkoxycarbonyl-5-alkyl-isoxazole, phthalimido and substituted phthalimido, and
X represents an oxygen atom, a sulphur atom, an imino group or an imino group substituted by a group selected from alkyl, alkoxy, hydroxy, amino, arylalkyloxy and aryloxy,
their enantiomers and diastereoisomers, and addition salts thereof with a pharmaceutically acceptable acid or base,
wherein, unless specified to the contrary,
- the term "substituted" associated with the systems thiophene, pyrazine, benzothiophene, 4-oxo[4H]benzopyran, pyrrole, pyrroline, pyrrolidine, piperidine, pyridine and phthalimido indicates that those systems are substituted by one or more groups selected from alkyl, alkoxy, trifluoromethyl, hydroxy, halogen, thiol and alkylthio,
- the terms "alkyl", "alkoxy" and "alkylene" denote linear or branched groups having from 1 to 6 carbon atoms,
- the term "aryl" denotes a phenyl or naphthyl radical, and
- the term "alkenylene" denotes an unsaturated, linear or branched chain having from 2 to 6 carbon atoms.

2. Compounds according to claim 1 of formula (IA): 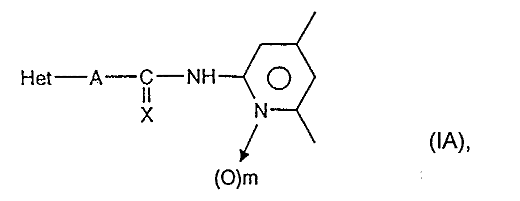 in which m represents 0 or 1, and
- either Het represents the thiophene group and A represents a methylene group, or
- Het represents the pyrazine group or the pyrazine group substituted by an alkyl group, and A is a single bond,
and X represents an oxygen atom, a sulphur atom, an imino group or an imino group substituted by a hydroxy group, a methoxy group, a methyl group, an amino group or by a benzyloxy group,
their enantiomers and diastereoisomers, and addition salts thereof with a pharmaceutically acceptable acid.

3. The compound according to claim 1 that is N-(4,6-dimethyl-pyridin-2-yl)-(thien-2-yl)-acetamide, its N-oxide and addition salts thereof with a pharmaceutically acceptable acid.

4. The compound according to claim 1 that is N-(4,6-dimethyl-pyridin-2-yl)-(thien-3-yl)-acetamide, its N-oxide and addition salts thereof with a pharmaceutically acceptable acid.

5. The compound according to claim 1 that is N-(4,6-dimethyl-pyridin-2-yl)-(pyrazin-2-yl)carbamidoxime, of formula : 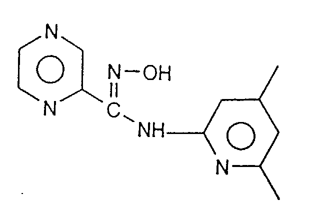 its N-oxide and addition salts thereof with a pharmaceutically acceptable acid.

6. The compound according to claim 1 that is N-(4,6-dimethyl-pyridin-2-yl)-O-methyl-(pyrazin-2-yl)carbamidoxime, of formula : 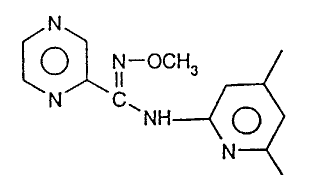 its N-oxide, the enantiomers thereof and addition salts thereof with a pharmaceutically acceptable acid.

7. The compound according to claim 1 that is N-(4,6-dimethyl-pyridin-2-yl)-(5-methylpyrazin-2-yl)thiocarboxamide, its N-oxide and addition salts thereof with a pharmaceutically acceptable acid.

8. The compound according to claim 1 that is N-(4,6-dimethyl-pyridin-2-yl)-(pyrazin-2-yl)carboxamidine, its N-oxide and addition salts thereof with a pharmaceutically acceptable acid.

9. The compound according to claim 1 that is N-methyl-N'-(4,6-dimethyl-pyridin-2-yl)-(2-pyrazin-2-yl)carboxamidine, its N-oxide and addition salts thereof with a pharmaceutically acceptable acid.

10. Process for the preparation of compounds of formula (I) according to claim 1, characterised in that there is used as starting material a compound of formula (1): 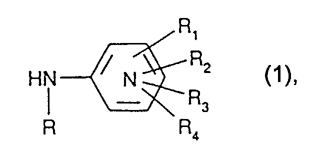 wherein R, R₁, R₂, R₃, R₄ and the ring 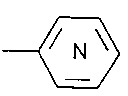 are as defined in claim 1, which is
• either condensed with a compound of formula (2) :
Het-A-COOH (2),
wherein Het and A have the same definitions as in claim 1,
to yield a compound of formula (I/a) : 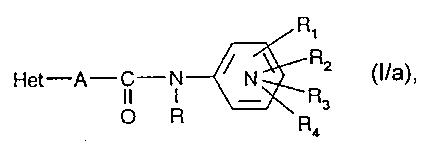 wherein Het, A, R, R₁, R₂, R₃ and R₄ and the ring 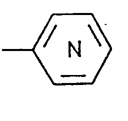 are as defined above,
which compound of formula (I/a) is treated with a thionating agent to yield a compound of formula (I/b) : 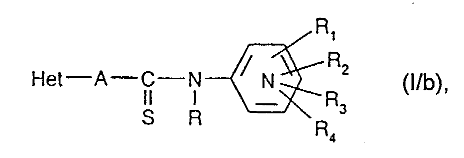 wherein Het, A, R, R₁, R₂, R₃ and R₄ and the ring 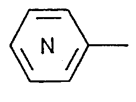 are as defined above, which compound of formula (I/b) is condensed with a compound of formula (3) : 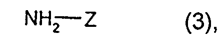 wherein Z represents a hydrogen atom, a hydroxy, alkyl, alkoxy, amino, arylalkyloxy or aryloxy group, to yield a compound of formula (I/c): 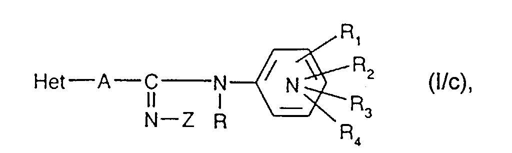 wherein Het, A, R, R₁, R₂, R₃, R₄, Z and the ring 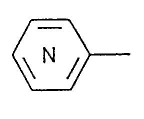 are as defined above,
• or reacted with a compound of formula (4) : 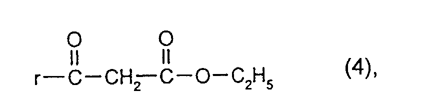 wherein r is an alkyl group,
to obtain compounds of formula (5) : 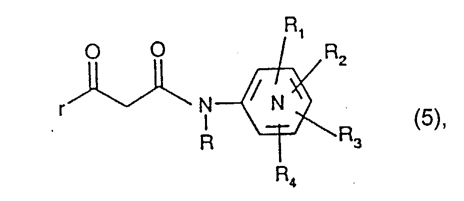 in which r, R, R₁, R₂, R₃ and R₄ are as defined above,
which are reacted with a compound of the formula Het-H, wherein Het is as defined in formula (I),
to obtain compounds of formula (lid), 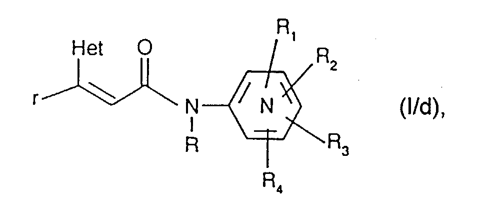 in which Het, r, R, R₁, R₂, R₃ and R₄ are as defined previously,
which compounds of formula (I/d) may, if desired, be reduced, by the action of sodium borohydride for example, to give compounds of formula (I/e) : 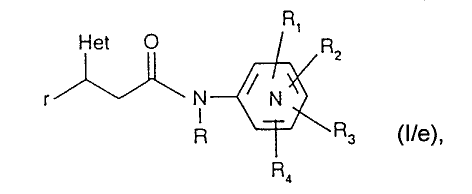 in which Het, r, R, R₁, R₂, R₃ and R₄ are as defined previously,
or which may, in the case where Het represents a pyrrolidine group, be reacted with alkyl chlorooximidoacetate of formula (6) : 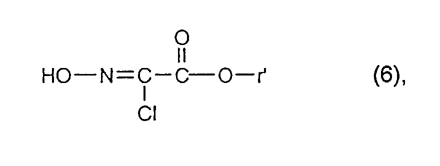 wherein r' is an alkyl group,
to give compounds of formula (I/f) : 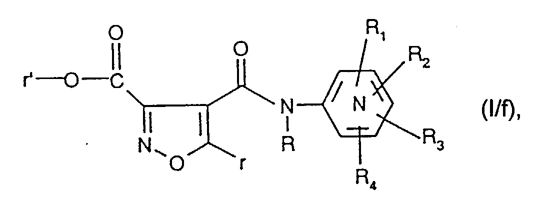 in which r, r', R, R₁, R₂, R₃ and R₄ are as defined previously,
which compounds of formula (I/f) may be reacted, if desired, with lithium hydroxide to obtain compounds of formula (I/g) : 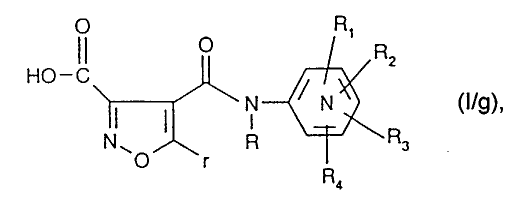 in which r, R, R₁, R₂, R₃ and R₄ are as defined previously,
which compounds of formulae (I/a), (I/b), (I/c), (I/d), (I/e), (I/f) and (I/g) may, if desired, be converted by the action of hydrogen peroxide into N-oxide derivatives of pyridine,
the compounds of formulae (I/a), (I/b), (I/c), (I/d), (I/e), (I/f) and (I/g) and their N-oxides forming the totality of the compounds of formula (I) according to claim 1, the enantiomers and diastereoisomers of which compounds of formula I may be separated and which compounds of formula (I) may be converted into a salt by means of a pharmaceutically acceptable acid or base.

11. Pharmaceutical composition comprising as active ingredient at least one compound according to one of claims 1 to 9 in combination with one or more inert, non-toxic, pharmaceutically acceptable excipients or carriers.

12. Pharmaceutical composition according to claim 11 comprising at least one active ingredient according to one of claims 1 to 9 for use in the treatment of inflammatory disorders, inflammatory renal diseases, arthritis, psoriasis and eczema.
